# EUROPÄISCHE PATENTSCHRIFT

(11) **EP 1 972 203 B1**
(45) Veröffentlichungstag und Bekanntmachung des Hinweises auf die Patenterteilung: **16.05.2012**
(21) Anmeldenummer: 08152668.3
(22) Anmeldetag: 13.03.2008
(51) Int. Cl.: A23L 1/22, C07C 49/825, C07C 49/835, C07C 49/84

(54) **Verwendung von 4-Hydroxychalkonderivaten zur Maskierung eines unangenehmen Geschmacks**
Use of 4-hydroxychalkon derivatives for masking an unpleasant taste
Utilisation de dérivés de 4-hydroxychalcone pour masquer un goût désagréable

(30) Priorität: 13.03.2007 US 894557 P
(43) Veröffentlichungstag der Anmeldung: 24.09.2008
(73) Patentinhaber: Symrise AG, 37603 Holzminden (DE)
(72) Erfinder: Ley, Jakob, 37603 Holzminden (DE); Paetz, Susanne, 37671 Höxter (DE); Krammer, Gerhard, 37603 Holzminden (DE); Riess, Thomas, 37603 Holzminden (DE)
(74) Vertreter: Eisenführ, Speiser & Partner

(56) Entgegenhaltungen:
- WO-A-2006/106023
- WO-A-2007/107596
- JP-A- 10 276 712
- US-A- 3 976 790
- YAMATO M ET AL: "Chemical structure and sweet taste of isocoumarins and related compounds. Synthesis of 5-hydroxyflavanones and related dihydrochalcones" CHEMICAL AND PHARMACEUTICAL BULLETIN 1977 JP, Bd. 25, Nr. 6, 1977, Seiten 1484-1486, XP002489894 ISSN: 0009-2363
- YAMATO M ET AL: "Chemical structure and sweet taste of isocoumarins and related compounds. X. Syntheses of sweet 5-hydroxyflavanones and related dihydrochalcones" CHEMICAL AND PHARMACEUTICAL BULLETIN 1978 JP, Bd. 26, Nr. 8, 1978, Seiten 2321-2327, XP002489895 ISSN: 0009-2363
- DUBOIS G E ET AL: "Dihydrochalcone Sweeteners. A study of the Atypical Temporal Phenomena" JOURNAL OF MEDICINAL CHEMISTRY, USAMERICAN CHEMICAL SOCIETY. WASHINGTON, Bd. 24, 1. Januar 1981 (1981-01-01), Seiten 408-428, XP002345673 ISSN: 0022-2623

## Beschreibung

Die Erfindung betrifft die Verwendung bestimmter 4-Hydroxychalkonderivate, deren Salzen und deren Gemischen zur Maskierung oder Verminderung unangenehmer Geschmackseindrücke, insbesondere bitterer, adstringierender und/oder metallischer Geschmackseindrücke. Ferner betrifft die Erfindung bestimmte Zubereitungen, die einen wirksamen Gehalt an den bezeichneten 4-Hydroxychalkonderivaten, deren Salzen oder deren Gemische enthalten.

Nahrungs- oder Genussmittel enthalten häufig verschiedene Bitterstoffe, die zwar einerseits in Maßen erwünscht und charakteristisch sind (z.B. Coffein in Tee oder Kaffee, Chinin in sogenannten Bitter-Lemon-Getränken, Hopfenextrakte in Bier), andererseits den Wert aber auch stark mindern können (z.B. Flavonoidglycoside und Limonoide in Zitrus-Säften, bitterer Nachgeschmack vieler künstlicher Süßstoffe wie Aspartam oder Saccharin, hydrophobe Aminosäuren und/oder Peptide in Käse).

Zur Senkung des natürlichen Gehalts an Bitterstoffen ist daher oft eine nachträgliche Behandlung nötig, beispielsweise extraktiv wie bei der Entcoffeinierung von Tee bzw. Kaffee, oder enzymatisch, z.B. Behandlung von Orangensaft mit einer Glycosidase zur Zerstörung des bitteren Naringins oder Einsatz von speziellen Peptidasen bei der Reifung von Käse. Diese Behandlung ist belastend für das Produkt, erzeugt Abfallstoffe und bedingt z.B. auch Lösungsmittelreste und andere Rückstände (Enzyme) in den Produkten.

Daher ist es wünschenswert, Stoffe zu finden, die unangenehme Geschmackseindrücke, insbesondere bittere, adstringierende und/oder metallische Geschmackseindrücke, wirkungsvoll unterdrücken, oder zumindest vermindern können.

Besonders wichtig ist die Unterdrückung des bitteren Geschmacks bei vielen pharmazeutischen Wirkstoffen, da dadurch die Bereitschaft der Patienten, insbesondere bei bitterempfindlichen Patienten wie Kindern, die den Wirkstoff enthaltende Zubereitung oral zu sich zu nehmen, deutlich erhöht werden kann. Viele pharmazeutische Wirkstoffe, beispielsweise Aspirin, Salicin, Paracetamol, Ambroxol oder Chinin, um nur eine sehr kleine Auswahl zur Verdeutlichung zu nennen, haben einen ausgeprägten bitteren, adstringierenden und/oder metallischen Geschmack und/oder Nachgeschmack.

Ein zunehmend wichtiger werdendes Maskierungsprobleme stellt der vermehrte Einsatz von Kaliumsalzen anstelle von Natriumsalzen dar. Insbesondere wird Natriumchlorid inzwischen häufig in würzigen Anwendungen zumindest partiell durch Kaliumchlorid ersetzt, das zwar als ein salzig schmeckendes Salz eingesetzt wird, aber einige zusätzliche, unangenehme Geschmackseindrücke hervorruft und insbesondere als bitter und metallisch schmeckend beschrieben wird.

Zwar kennt man einige Stoffe, die den bitteren Geschmack partiell unterdrücken können, doch zeigen viele in der Anwendung starke Limitationen.

In US 5,637,618 wird ein bitterer Geschmack, insbesondere auch der bittere Geschmack von Kaliumsalzen, mit Hilfe von Lactisol [20-(4-Methoxyphenyl)milchsäure] reduziert. Dieser Inhibitor zeigt aber gleichzeitig eine starke Inhibition des süßen Geschmackseindrucks (vgl. US 5,045,336), was die Anwendbarkeit stark einschränkt.

2,4-Dihydroxybenzoesäure-Kaliumsalz wird in US 5,643,941 (Tabelle Spalte 3, Zeile 18) als Maskierer für den bitteren Geschmack von Kaliumchlorid bzw. Kaliumsalzen beschrieben, kann aber z.B. den Geschmack von Coffein nicht unterdrücken.

Gemäß GB 2,380,936 wird die Unterdrückung des Geschmacks bitterer Pharmazeutika mit Ingwerextrakten erreicht. Für eine Vielzahl von Anwendungen sind Ingwerextrakte jedoch nicht geeignet, da sie einen starken Aromaeindruck hervorrufen und/oder ihre häufig zu findende Schärfe störend wirkt.

In US-A 5,580,545 werden zwar für einige Flavone (2-Phenylchrom-2-en-4-one) geschmacksverändernde Eigenschaften beschrieben, eine Bitter-reduzierende oder -unterdrückende Wirkung wurde aber nicht gefunden.

In US 2002 177,576 wird die Unterdrückung eines bitteren Geschmacks durch Nucleotide, beispielsweise Cytidin-5'-monophosphate (CMP) beschrieben. Die stark polaren und daher nur in stark polaren Lösungsmitteln verwendbaren Verbindungen sind aber in vielen fetthaltigen Nahrungsmitteln nur sehr eingeschränkt verwendbar. Zudem ist die Verfügbarkeit solcher Stoffe auf Grund ihrer aufwändigen chemischen Synthese stark limitiert.

In US 2006/286276 werden Mischungen von Nucleotiden, beispielsweise Adenosin-5-monophosphat (AMP) mit der Aminosäure Taurin beschrieben, die in Kochsalz-reduzierten Anwendungen, die gleichzeitig einen erhöhten Anteil Kaliumchlorid haben, den bitteren und metallischen Geschmack des Kaliumsalzes zumindest teilweise senken können.

In US 2002/0188019 werden Hydroxyflavanone als wirksame Bitter-Maskierer beschrieben, welche aber nur schwer synthetisch zugänglich und nicht in größeren Mengen preiswert verfügbar sind.

Die Natriumsalze Natriumchlorid, Natriumcitrat, Natriumacetat und Natriumlactat zeigen einen bitter-maskierenden Effekt gegen viele Bitterstoffe (z.B. Nature, 1997, Bd. 387, S. 563); allerdings kann die Aufnahme größerer Mengen Natriumionen z.B. zu Herz-Kreislauferkrankungen führen. Eine signifikante Bitter-maskierende Wirkung tritt zudem nachteiligerweise erst bei relativ hohen Natriumkonzentrationen ein (ab ca. 0,1 M), was z.B. einem in der Regel inakzeptabel hohen Anteil von ca. 0,6 Gew.-% NaCl in der Endanwendung entspricht (vgl. R.S.J. Keast, P.A.S. Breslin und G.K. Beauchamp, Chimia 2001, 55(5), 441-447).

In WO 00/21390 wird Polyglutaminsäure als Bitterkeit-maskierendes Agens beschrieben; dabei werden relativ hohe Konzentrationen im Bereich um 1 Gew.-% benötigt.

Ein Lipoprotein bestehend aus β-Lactoglobulin und Phosphatidinsäure, zeigt ebenfalls einen Bitter-maskierenden Effekt (EP-A 635 218). Solche Polymere sind allerdings schwierig zu charakterisieren und zu standardisieren und zeigen einen ausgeprägt seifigen Nebengeschmack.

Das Flavonglycosid Neodiosmin [5,7-Dihydroxy-2-(4-methoxy-3-hydroxyphenyl)-7-O-neohesperidosyl-chrom-2-en-4-on] zeigt ebenfalls eine Bitter-maskierende Wirkung (US-A 4,154,862), zeichnet sich aber durch einen Disaccharid-Rest aus, der die Herstellung bzw. Isolierung und Anwendbarkeit der Substanz sehr erschwert.

In WO 2005/096841 wird γ-Aminobuttersäure als Bittermaskierer vorgeschlagen; diese ist zwar gut wirksam, bringt aber zusätzlich einen leicht sauren Geschmack in die Zubereitung. In WO 2006/024587 werden Hydroxybenzoesäurevanillylamide als Maskierer für bitteren Geschmack beschrieben; ebenso einige kurzkettige Gingerdionderivate nach WO 2007/003527 und Hydroxydeoxybenzoine nach WO 2006/106023, die aber alle nicht in natürlichen Quellen vorkommen.

Es war die primäre Aufgabe der vorliegenden Erfindung, (a) Stoffe zu finden, die zur Maskierung oder Verminderung des unangenehmen Geschmackseindrucks unangenehm schmeckender Stoffe geeignet sind (und vorzugsweise insbesondere einen Bitter-maskierenden Effekt gegen eine Vielzahl von Bitterstoffen zeigen), (b) breit anwendbar sind, (c) leicht zugänglich sind und (d) im Idealfall natürlich vorkommen.

Die gestellte Aufgabe wird erfindungsgemäß gelöst durch die Verwendung
- eines 4-Hydroxychalkonderivats der Formel (I) 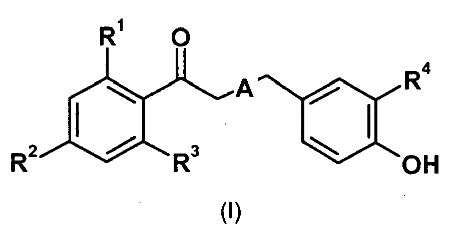 wobei
   - A: eine Einfach- oder Doppelbindung darstellen kann,
   - R¹, R² und R³,: unabhängig voneinander, jeweils H, OH oder (vorzugsweise C₁-C₄-) Alkoxy bedeuten können, mit der Maßgabe, dass mindestens einer der Reste R¹ bis R³ OH bedeutet,
   - und R⁴: H, OH oder (vorzugsweise C₁-C₄-) Alkoxy bedeutet,
   und/oder
- eines Salzes eines solchen 4-Hydroxychalkonderivats der Formel (I),
- einer Mischung umfassend oder bestehend aus zwei oder mehr unterschiedlichen 4-Hydroxychalkonderivaten der Formel (I), wobei R¹, R², R³ und R⁴ jeweils die oben angegebene Bedeutung haben,
- einer Mischung umfassend oder bestehend aus Salzen von zwei oder mehr unterschiedlichen 4-Hydroxychalkonderivaten der Formel (I), wobei R¹, R², R³ und R⁴ jeweils die oben angegebene Bedeutung haben,
oder
- einer Mischung umfassend oder bestehend aus
   einem 4-Hydroxychalkonderivat der Formel (I) oder zwei oder mehr unterschiedlichen 4-Hydroxychalkonderivaten der Formel (I), wobei R¹, R², R³ und R⁴ jeweils die oben angegebene Bedeutung haben, und
   einem Salz eines 4-Hydroxychalkonderivats der Formel (I) oder zwei oder mehr Salzen von unterschiedlichen 4-Hydroxychalkonderivaten der Formel (I), wobei R¹, R², R³ und R⁴ jeweils die oben angegebene Bedeutung haben,
zum Verändern, Maskieren oder Vermindern des unangenehmen Geschmackseindrucks eines unangenehm schmeckenden Stoffes, insbesondere eines bitteren Geschmacks bzw. bitteren Nachgeschmacks.

Unangenehm schmeckende Stoffe im Sinne der Erfindung sind:
(a) Stoffe, die bitter, adstringierend, pappig, staubig, trocken, mehlig, ranzig und/oder metallisch schmecken sowie
(b) Stoffe, die einen bitteren, adstringierenden, pappigen, staubigen, trockenen, mehligen, ranzigen oder metallischen Nachgeschmack haben.

Die vorgenannten unangenehm schmeckenden Stoffe können noch weitere, in der Regel nicht unangenehme Geschmacks- und/oder Geruchsqualitäten besitzen. Als weitere, im Sinne der vorliegenden Erfindung nicht unangenehme Geschmacksqualitäten sind z.B. die Eindrücke würzig, umami, süß, salzig, sauer, scharf, kühlend, wärmend, brennend oder kribbelnd zu nennen.

Stoffe, die bitter, adstringierend, pappig, staubig, trocken, mehlig, ranzig oder metallisch schmecken, sind beispielsweise: Xanthinalkaloide, Xanthine (Coffein, Theobromin, Theophyllin und Methylxanthine), Alkaloide (Chinin, Brucin, Strychnin, Nicotin), phenolische Glycoside (z.B. Salicin, Arbutin), Flavonoidglycoside (z.B. Neohespereidin, Hesperidin, Naringin, Quercitrin, Rutin), bitter schmeckende Chalcone oder Chalconglycoside (z.B. Phloridzin), hydrolisierbare Tannine (Gallus- oder Elagsäureester von Kohlenhydraten, z.B. Pentagalloylglucose), nichthydrolisierbare Tannine (ggfs. galloylierte Catechine oder Epicatechine und deren Oligomeren, z.B. Proanthyocyanidine oder Procyanidine, Thearubigenin), Flavone (z.B. Quercetin, Taxifolin, Myricetin), Phenole wie z.B. Salicin, Polyphenole (z.B. γ-Oryzanol, Kaffeesäure oder deren Ester), terpenoide Bitterstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (insbesondere Kalium-, Magnesium- und Calciumsalze, Kaliumchlorid, Kaliumgluconat, Kaliumcarbonat, Kaliumsulfat, Kaliumlactat, Kaliumglutamat, Kaliumsuccinat, Kaliummalat, Natriumsulfat, Magnesiumsulfat), pharmazeutische Wirkstoffe (z.B. Fluorchinolon-Antibiotika, Paracetamol, Aspirin, β-Lactam-Antibiotika, Ambroxol, Propylthiouracil [PROP], Guaifenesin), Vitamine (beispielsweise Vitamin H, Vitamine aus der B-Reihe wie Vitamin B1, B2, B6, B12, Niacin, Panthotensäure), Denatoniumbenzoat, Sucraloseoctaacetat, Eisensalze, Aluminiumsalze, Zinksalze, Harnstoff, ungesättigte Fettsäuren, insbesondere ungesättigte Fettsäuren in Emulsionen, bitter schmeckende Aminosäuren (z.B. Leucin, Isoleucin, Valin, Tryptophan, Prolin, Histidin, Tyrosin, Lysin oder Phenylalanin) und bitter schmeckende Peptide (insbesondere Peptide mit einer Aminosäure aus der Gruppe Leucin, Isoleucin, Valin, Tryptophan, Prolin oder Phenylalanin am N- oder C-Terminus). Bevorzugt sind dabei als Stoffe, die bitter, adstringierend, pappig, staubig, trocken, mehlig, ranzig oder metallisch schmecken, Coffein, Theobromin und Theophyllin, Chinin, Salicin, Arbutin, Neohesperidin, Naringin, Quercitrin, Rutin, Phloridzin, Gallus- oder Elagsäureester von Kohlenhydraten (z.B. Pentagalloylglucose), ggfs. galloylierte Catechine oder Epicatechine, Proanthyocyanidine oder Procyanidine, Thearubigenin, Quercetin, Taxifolin, Myricetin, γ-Oryzanol, Kaffeesäure oder deren Ester (z.B. Chlorogensäure und Isomere), Limonoide wie Limonin oder Nomilin aus Zitrusfrüchten, Lupolone und Humolone aus Hopfen, Absinthin aus Wermut, Amarogentin aus Enzian, metallische Salze (insbesondere Kalium-, Magnesium- und Calciumsalze, Kaliumchlorid, Kaliumgluconat, Kaliumcarbonat, Kaliumsulfat, Kaliumlactat, Kaliumglutamat, Kaliumsuccinat, Kaliummalat, Natriumsulfat, Magnesiumsulfat) und pharmazeutische Wirkstoffe (z.B. Fluorchinolon-Antibiotika, Paracetamol, Aspirin, β-Lactam-Antibiotika, Ambroxol, Propylthiouracil [PROP], Guaifenesin).

Stoffe, die einen bitteren, adstringierenden, pappigen, kalkigen, staubigen, trockenen, mehligen, ranzigen oder metallischen Nachgeschmack haben, können Aroma- oder Geschmackstoffe mit einem nicht unangenehmen Primärgeschmack (beispielsweise süß, salzig, würzig, sauer) und/oder -geruch sein, und z.B. zur Gruppe der Süßstoffe, Zuckeraustauschstoffe oder der Aromastoffe gehören. Beipielsweise seien genannt: Kaliumsalze (insbesondere Kaliumchlorid, Kaliumgluconat, Kaliumcarbonat, Kaliumsulfat, Kaliumlactat, Kaliumglutamat, Kaliumsuccinat, Kaliummalat), Aspartam, Neotam, Superaspartam, Saccharin, Sucralose, Tagatose, Monellin, Stevioside, Rebaudioside, Hernandulcin, Thaumatin, Miraculin, Glycyrrhizin, Glycyrrhetinsäure oder deren Derivate, Cyclamat oder die pharmazeutisch akzeptablen Salze der vorgenannten Verbindungen.

Besonders bevorzugt werden die erfindungsgemäßen 4-Hydroxychalkonderivate der Formel (I), ihre Salze und Gemische verwendet zum Verändern, Verringern oder Maskieren eines bitteren Geschmacks.

Bevorzugt ist die Verwendung der 4-Hydroxychalkonderivate der Formel (I) 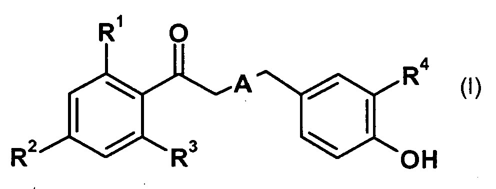 wobei
- A: eine Einfachbindung darstellt,
und
- R¹: OH bedeutet
und
- R² und R³,: unabhängig voneinander, H oder OH bedeuten können,
und
- R⁴: ein H oder Methoxy bedeutet,
und/oder deren Salzen und Mischungen wie oben beschrieben.

Ebenso bevorzugt ist die Verwendung der 4-Hydroxychalkonderivate der Formel (I) 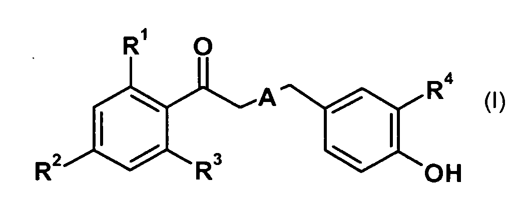 wobei
- A: eine Einfachbindung darstellt,
und
- R²: OH bedeutet
und
- R¹ und R³ H: bedeuten,
und
- R⁴: ein H oder Methoxy bedeutet,
und/oder deren Salzen und Mischungen wie oben beschrieben.

Besonders bevorzugt als 4-Hydroxychalkonderivate der Formel (I) sind:
2',4-Dihydroxydihydrochalkon oder 3-(4-Hydroxyphenyl)-1-(2-hydroxyphenyl)propan-1-on (Verbindung 1)
2',4,4'-Trihydroxydihydrochalkon oder 3-(4-Hydroxyphenyl)-1-(2,4-dihydroxyphenyl)propan-1-on (Davidigenin, Verbindung 2)
2',4,6'-Trihydroxydihydrochalkon oder 3-(4-Hydroxyphenyl)-1-(2,6-dihydroxyphenyl)propan-1-on (Verbindung 3)
2',4,4',6'-Tetrahydroxydihydrochalkon oder 3-(4-Hydroxyphenyl)-1-(2,4,6-trihydroxyphenyl)propan-1-on (Phloretin, Verbindung 4)
2',4-Dihydroxy-3-methoxydihydrochalkon oder 3-(4-Hydroxy-3-methoxyphenyl)-1-(2-hydroxyphenyl)propan-1-on (Verbindung 5)
2',4,4'-Trihydroxy-3-methoxydihydrochalkon oder 3-(4-Hydroxy-3-methoxyphenyl)-1-(2,4-dihydroxyphenyl)propan-1-on (Verbindung 6)
2',4,6'-Trihydroxy-3-methoxydihydrochalkon oder 3-(4-Hydroxy-3-methoxyphenyl)-1-(2,6-dihydroxyphenyl)propan-1-on (Verbindung 7)
2',4,4',6'-Tetrahydroxy-3-methoxydihydrochalkon oder 3-(4-Hydroxy-3-methoxyphenyl)-1-(2,4,6-trihydroxyphenyl)propan-1-on (Verbindung 8)
2',3,4,4',6'-Tetrahydroxydihydrochalkon oder 3-(3,4-Dihydroxyphenyl)-1-(2,4,6-trihydroxyphenyl)propan-1-on (3-Hydroxyphloretin, Verbindung 9)
4,4'-Dihydroxy-3-methoxy-dihydrochalkon oder 3-(4-Hydroxy-3-methoxyphenyl)-1-(4-hydroxyphenyl)propan-1-on (Verbindung 10)
und deren Salze und Gemische wie oben beschrieben.

Zwar sind Verbindungen der allgemeinen Formel (I), wenn A eine Einfachbindung bezeichnet, als solche bereits in der WO 2007/014879 erwähnt; ihre Verwendbarkeit zum Verändern, Maskieren oder Vermindern des unangenehmen Geschmacks eines unangenehm schmeckenden Stoffes war jedoch nicht bekannt. Die Verbindungen sind nur zusammen mit Hesperetin beschrieben, das zum Verstärken eines süßen Geschmacks eingesetzt werden sollte.

Die Verbindungen 1 bis 10 sind in der folgenden Abbildung noch einmal zur Verdeutlichung aufgeführt (an Verbindung 1 ist exemplarisch das allgemeine Numerierungsschema für Dihydrochalkone angegeben):

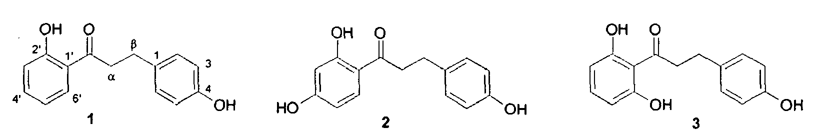

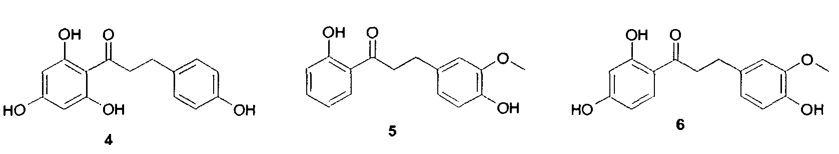

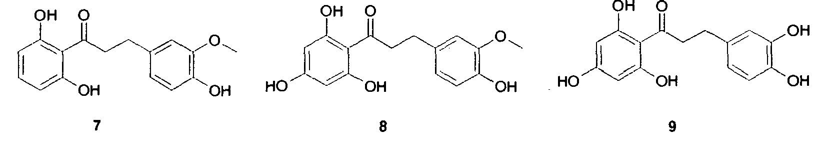

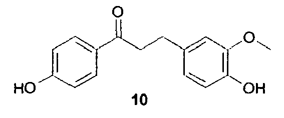

In Salzen einer erfindungsgemäß zu verwendenden Verbindung der obigen Formel (I) (wobei hinsichtlich der bevorzugten Bedeutungen der Reste und Variablen der zuvor Gesagte weiterhin gilt) sind eine, mehrere oder sämtliche Hydroxy-Gruppen der Verbindung (I) deprotoniert. Es liegt dann eine entsprechende Menge von Gegenkationen vor, wobei diese vorzugsweise ausgewählt sind aus der Gruppe bestehend aus: einfach positiv geladene Kationen der ersten Haupt- und Nebengruppe, Ammoniumionen, Trialkylammoniumionen, zweifach positiv geladene Kationen der zweiten Haupt- und Nebengruppe sowie dreifach positiv geladene Kationen der dritten Haupt- und Nebengruppe, und deren Mischungen.

Es versteht sich, dass die Zahl der Hydroxy-Gruppen im zugrunde liegenden 4-Hydroxychalkonderivaten entscheidend ist für den maximalen Deprotonierungsgrad und somit auch für die Menge vorhandener Gegenkationen. Sind beispielsweise insgesamt zwei Hydroxy-Gruppen im zugrundeliegenden 4-Hydroxychalkonderivaten vorhanden, liegt bei vollständiger Deprotonierung der Hydroxy-Gruppen ein zweifach negativ geladenes Anion vor, sodass eine entsprechende Zahl von positiven Ladungen durch das bzw. die Gegenkationen bereitgestellt werden muss.

Besonders bevorzugte Kationen sind Na⁺, K⁺, NH₄⁺, Ca²⁺, Mg²⁺, Al³⁺ und Zn²⁺.

Selbstverständlich können die verschiedenen erfindungsgemäßen 4- Hydroxychalkonderivate und deren Salze jeweils alleine oder als Gemische erfindungsgemäß verwendet werden.

Bevorzugt ist auch eine erfindungsgemäße Verwendung, bei der neben einer Verbindung der Formel (I), einem entsprechenden Salz oder einem entsprechenden Gemisch eine weitere Substanz zum Maskieren oder Vermindern des unangenehmen Geschmackseindrucks eines unangenehm schmeckenden Stoffes vorhanden ist. Es liegt dann die Kombination von zumindest zwei Geschmackskorrigenzien vor.

Das bekannte Neohesperidindihydrochalcon zeigt einen bitter-reduzierenden Effekt, was aber vor allem auf die Eigenschaft als sehr starker Süßstoff zurückzuführen ist (vgl. Manufacturing Chemist 2000, Juli-Heft, S. 16-17) und in nichtsüßen Anwendungen auch störend wirkt. Aus eigenen Untersuchungen ergab sich jedoch, dass die erfindungsgemäß zu verwendenden 4-Hydroxydihydrochalkonderivate der Formel (I) oder deren Salze, wie beispielsweise Phloretin (Verbindung 4), verglichen mit typischen süß schmeckenden Stoffen nur eine sehr schwache Eigensüße aufweisen. Erfindungsgemäß werden die 4-Hydroxychalkonderivate der Formel (I) nicht als Süßungsmittel eingesetzt. Die 4-Hydroxychalkonderivate der Formel (I) besitzen zudem weitere schwache intrinsischen Geschmackseigenschaften, die nicht süß sind (vgl. sensorische Profilbeschreibung in den Beispielen 1 - 4). Ein bittermaskierender Effekt der erfindungsgemäßen, maximal schwach süßen 4-Hydroxychalkonderivate der Formel (I) oder deren Salze ist nicht vorbeschrieben.

Bezüglich des Vorkommens und der Synthese der erfindungsgemäß verwendeten 4-Hydroxychalkonderivate der Formel (I) sei auf die US 60/784,444 (jetzt WO 2007/107596) verwiesen. Dieses Dokument ist als Prioritätsdokument zur WO 2007/014879 durch Akteneinsicht zugänglich und wird hier zum Zwecke der vollständigen Offenbarung der vorliegenden Erfindung in Gänze in Bezug genommen.

Überraschenderweise wurde gefunden, dass die erfindungsgemäß verwendeten 4-Hydroxychalkonderivate der Formel (I) auch in sehr geringen Konzentrationen den unangenehmen Geschmackseindruck, insbesondere den bitteren Geschmackseindruck einer Vielzahl von Stoffen, insbesondere von Coffein, Theobromin und Theophyllin, Chinin, Salicin, Arbutin, Neohesperidin, Naringin, Quercitrin, Rutin, Phloridzin, Gallus- oder Elagsäureester von Kohlenhydraten (z.B. Pentagalloylglucose), ggfs. galloylierte Catechine oder Epicatechine, Proanthyocyanidine oder Procyanidine, Thearubigenin, Quercetin, Taxifolin, Myricetin, γ-Oryzanol, Kaffeesäure oder deren Ester (z.B. Chlorogensäure und Isomere), Limonoide wie Limonin oder Nomilin aus Zitrusfrüchten, Lupolone und Humolone aus Hopfen, Absinthin aus Wermut, Amarogentin aus Enzian, metallische Salze (insbesondere Kalium-, Magnesium- und Calciumsalze, Kaliumchlorid, Kaliumgluconat, Kaliumcarbonat, Kaliumsulfat, Kaliumlactat, Kaliumglutamat, Kaliumsuccinat, Kaliummalat, Natriumsulfat, Magnesiumsulfat) und pharmazeutische Wirkstoffe (z.B. Fluorchinolon-Antibiotika, Paracetamol, Aspirin, β-Lactam-Antibiotika, Ambroxol, Propylthiouracil [PROP], Guaifenesin) reduzieren oder sogar vollständig unterdrücken können, wobei es besonders vorteilhaft ist, dass die erfindungsgemäß verwendeten 4-Hydroxychalkonderivate nahezu keinen Eigengeschmack besitzen und die weiteren, in der Regel nicht unangenehmen Geschmacksqualitäten nicht negativ, insbesondere den süßen Geschmack von süßen Stoffen sogar positiv beeinflussen.

Überraschenderweise wurde ferner gefunden, dass die erfindungsgemäß verwendeten 4-Hydroxychalkonderivate der Formel (I) in Kombination mit einem oder mehreren Lactonen, vorzugsweise Lactonen mit einem 5- oder 6-gliedrigen Ring, insbesondere bevorzugt in Kombination Nonenolid und/oder 4-Methyl-5-hydroxy-hexansäurelacton (4-Methyl-delta-hexalacton), besonders gut den bitteren Geschmack reduzieren, vor allem von Kaliumsalzen (insbesondere Kaliumchlorid, Kaliumgluconat, Kaliumcarbonat, Kaliumsulfat, Kaliumlactat, Kaliumglutamat, Kaliumsuccinat oder Kaliummalat), speziell von Kaliumchlorid. Dabei wird tatsächlich der bittere Geschmackseindruck der Kaliumsalze verringert und nicht etwa durch einen anderen stärkeren (z. B. süßen) Geschmackseindruck überlagert.

Nonenolid kann dabei bevorzugt als (R)- oder (S)-Enantiomer oder in racemischer Form eingesetzt werden. 4-Methyl-5-hydroxy-hexansäurelacton kann dabei syn-oder anti-konfiguriert sein, als (R,R)-konfiguriertes Enantiomer, (R,S)-konfiguriertes Enantiomer, (S,R)-konfiguriertes Enantiomer, (S,S)-konfiguriertes Enantiomer oder als beliebiges Gemisch der Enantiomeren, insbesondere als Racemat, oder auch als beliebiges Gemisch der entsprechenden Diastereomeren, vorliegen.

Wie bereits erwähnt, betrifft ein Aspekt der vorliegenden Erfindung die Verwendung eines 4-Hydroxychalkonderivats der Formel (I) bzw. eines entsprechenden Salzes oder Gemisches zur Maskierung oder Verminderung des unangenehmen Geschmackseindruckes eines unangenehm schmeckenden Stoffes, d. h. als Geschmackskorrigenz. Vorzugsweise werden das erfindungsgemäß zu verwendende 4-Hydroxychalkonderivat der Formel (I), das Salz oder das Gemisch in einer zur oralen Aufnahme hergerichteten pharmazeutischen Zubereitung, einer der Ernährung, der Mundpflege oder dem Genuss dienenden Zubereitung eingesetzt, wobei die Zubereitung üblicherweise einen oder mehrere unangenehm schmeckende Stoffe umfasst.

Soweit die vorliegende Erfindung Zubereitungen betrifft, sind dabei vorzugsweise solche Zubereitungen ausgenommen, die Phloretin und a) Hesperetin und/oder b) Phloridzin oder ein anderes Phloretinglycosid enthalten. Ausgenommen sind daher bevorzugt diejenigen Zubereitungen, die die erfindungsgemäßen 4-Hydroxychalkonderivate der Formel (I) natürlicherweise enthalten und denen die 4-Hydroxychalkonderivate der Formel (I) nicht aus anderen Quellen (synthetisch oder isoliert aus natürlichen Quellen) über das natürliche Maß hinaus zugesetzt werden. Hierunter fallen insbesondere viele Apfel-haltige Zubereitungen *(Malus* ssp.).

Wenn die erfindungsgemäße Zubereitung einen oder mehrere süße Stoffe besitzt, so ist ebenfalls bevorzugt, dass die Süße der erfindungsgemäßen Zubereitung maximal 1 % Sucrose-Äquivalente besitzt. Eine solche erfindungsgemäße Zubereitung besitzt dann eine Süße, die nicht größer ist als die einer 1 %igen Sucrose-Lösung. Solche Lösungen werden üblicherweise als nicht einmal schwach süß schmeckend empfunden. Insbesondere bei diesen Zubereitungen geht das Verringern, Verändern oder Maskieren eines unangenehmen, insbesondere eines bitteren, Geschmacks eines unangenehm schmeckenden Stoffes erkennbar nicht zurück auf eine Überdeckung des unangenehmen Geschmacks durch eine sehr starke Süße der Zubereitung.

Erfindungsgemäße Zubereitungen umfassen vorzugsweise 0,000001 Gew.-% bis 95 Gew.-%, bezogen auf das Gesamtgewicht der Zubereitung, einer erfindungsgemäß zu verwendenden Verbindung der Formel (I) (siehe oben), eines entsprechenden Salzes oder Gemisches. Daneben sind üblicherweise ein oder mehrere unangenehm schmeckende Stoffe vorhanden.

Besonders bevorzugt sind erfindungsgemäße Zubereitungen, die zumindest einen unangenehm schmeckenden Stoff umfassen, wobei die Menge des unangenehm schmeckenden Stoffes ausreicht, um in einer Vergleichszubereitung, die kein 4-Hydroxychalkonderivat der Formel (I), Salz oder Gemisch eines solchen 4-Hydroxychalkonderivats umfasst, aber ansonsten identisch zusammengesetzt ist, als unangenehmer Geschmack wahrgenommen zu werden, und die Menge des 4-Hydroxychalkonderivats der Formel (I), Salz oder des Gemisch eines solchen 4-Hydroxychalkonderivats in der Zubereitung ausreicht, um im Vergleich mit der Vergleichszubereitung den unangenehmen Geschmackseindruck des unangenehm schmeckenden Stoffes sensorisch zu verändern, zu maskieren oder zu vermindern.

Erfindungsgemäße Zubereitungen können als Halbfertigware, als Riech-, Aroma- oder Geschmacksstoffkomposition oder als Würzmischung vorliegen.

Der Ernährung oder dem Genuss dienende Zubereitungen im Sinne der Erfindung sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, eingekochte Gemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Fest-stoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende Zubereitungen im Sinne der Erfindung sind insbesondere Mund- und/oder Zahnpflegemittel wie Zahnpasten, Zahngele, Zahnpulver, Mundwässer, Kaugummis und andere Mundpflegemittel.

Weitere übliche Wirk-, Grund-, Hilfs- und Zusatzstoffe für der Ernährung, der Mundpflege oder dem Genuss dienende können in Mengen von 5 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Die erfindungsgemäßen Zubereitungen, enthaltend eines oder mehrere der erfindungsgemäß verwendeten 4-Hydroxychalkonderivate der Formel (I) bzw. deren Salze oder Gemische, werden gemäß einer bevorzugten Ausgestaltung hergestellt, indem die 4-Hydroxychalkonderivate der Formel (I) bzw. deren Salze oder Gemische als Substanzen, als Lösung oder in Form eines Gemischs mit einem festen oder flüssigen Trägerstoff in eine der Ernährung, der Mundpflege oder dem Genuss dienende oder orale pharmazeutische Basis-Zubereitung eingearbeitet werden. Vorteilhafterweise können als Lösung vorliegende erfindungsgemäße Zubereitungen auch durch Sprühtrocknung in eine feste Zubereitung überführt werden.

Gemäß einer weiteren bevorzugten Ausführungsform werden zur Herstellung erfindungsgemäßer Zubereitungen das bzw. die erfindungsgemäß verwendeten 4-Hydroxychalkonderivat(e) der Formel (I) bzw. deren Salze oder Gemische und gegebenenfalls andere Bestandteile der erfindungsgemäßen Zubereitung in Form von Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten, Cellulose oder Cellulosederivaten (z.B. Hydroxypropylcellulose), anderen Polysacchariden (z.B. Alginat), natürlichen Fetten, natürlichen Wachsen (z.B. Bienenwachs, Carnaubawachs) oder aus Proteinen, z.B. Gelatine, eingearbeitet.

In einem weiteren bevorzugten Herstellungsverfahren werden die 4-Hydroxychalkonderivate der Formel (I) bzw. deren Salze oder Gemische vorher mit einem oder mehreren geeigneten Komplexbildnern, beispielsweise mit Cycloglycanen, z.B. Cyclofructanen, Cyclodextrinen oder Cyclodextrinderivaten, bevorzugt α-, γ- und β-Cyclodextrin, komplexiert und in dieser komplexierten Form eingesetzt.

Besonders bevorzugt ist eine erfindungsgemäße Zubereitung, bei der die Matrix so gewählt wird, dass die 4-Hydroxychalkonderivate der Formel (I) verzögert von der Matrix freigegeben werden, so dass man eine langanhaltende Wirkung erhält.

Als weitere Bestandteile für erfindungsgemäße Zubereitungen können übliche Grund-, Hilfs- und Zusatzstoffe für Nahrungs- oder Genussmittel verwendet werden. Einige dieser Substanzen besitzen einen unangenehmen Geschmack im Sinne der eingangs gegebenen Definition.

Beispiele üblicher Grund-, Hilfs- und Zusatzstoffe für erfindungsgemäße Zubereitungen sind Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nichtproteinogene Aminosäuren und verwandte Verbindungen (z.B. Taurin), Peptide, native oder prozessierte Proteine (z.B. Gelatine), Enzyme (z.B. Peptidasen), Nukleinsäuren, Nucleotide, andere als die erfindungsgemäß verwendeten Geschmackskorrigenzien für unangenehme Geschmackseindrücke (4-Hydroxychalkonderivate der Formel (I) und Lactone wie oben beschrieben), Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), zusätzliche Bitterstoffe (z.B. Chinin, Coffein, Limonin, Amarogentin, Humolone, Lupolone, Catechine, Tannine), Süßstoffe (z.B. Saccharin, Cyclamat, Aspartam, Neotam), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal (das heißt scharfe, stechende, prickelnde, kratzende, adstringierende, Wärme- oder Kälte-Effekte verursachende) wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Basis für die Mundpflege dienende Zubereitungen), die die erfindungsgemäß zu verwendenden 4-Hydroxychalkonderivate der Formel (I), deren Salze oder Gemische enthalten, umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, andere Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für die Mundpflege dienende Zubereitungen), welche erfindungsgemäß zu verwendende 4-Hydroxychalkonderivate, deren Salze oder Gemische enthalten, umfassen im allgemeinen eine Kaugummibase, d.h. eine beim Kauen plastisch werdende Kaumasse, Zucker verschiedener Arten, Zuckeraustauschstoffe, Süßstoffe, Zuckeralkoholen, andere Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), die im vorherigen Abschnitt genannten Kühlwirkstoffe, Feuchthaltemittel, Verdicker, Emulgatoren, Aromen und Stabilisatoren oder Geruchskorrigentien.

Bevorzugt können die erfindungsgemäßen Zubereitungen auch eine Aromakomposition enthalten, um den Geschmack und/oder Geruch der Zubereitung abzurunden und zu verfeinern. Geeignete Aromakompositionen enthalten z.B. synthetische, natürliche oder naturidentische Aroma-, Riech- und Geschmacksstoffe sowie geeignete Hilfs- und Trägerstoffe.

Erfindungsgemäße Zubereitungen, die als Halbfertigwaren dienen, enthalten in der Regel 0,0001 Gew.-% bis 95 Gew.-%, bevorzugt 0,001 bis 80 Gew.-%, insbesondere aber 0,01 Gew.-% bis 50 Gew.-%, bezogen auf das Gesamtgewicht der Zubereitung, an erfindungsgemäß zu verwendenden 4-Hydroxychalkonderivaten der Formel (I), deren Salzen oder deren Gemischen. Erfindungsgemäße Zubereitungen, die als Halbfertigwaren vorliegen, können zur Maskierung oder Verminderung des unangenehmen Geschmackseindrucks von Fertigware-Zubereitungen dienen, die unter Verwendung der Halbfertigware-Zubereitung hergestellt werden.

In einer besonders bevorzugten Ausführung der Erfindung werden die erfindungsgemäßen 4-Hydroxychalkonderivate der Formel (I), deren Salze oder Gemische in den erfindungsgemäßen Zubereitungen in Kombination mit zumindest einer weiteren (anderen) Substanz zum Verändern, Maskieren oder Vermindern des unangenehmen Geschmackseindrucks eines unangenehm schmeckenden Stoffes verwendet. Auf diese Weise kann eine besonders wirksame Maskierung erreicht werden.

Die weiteren Geschmackskorrigenzien können aus der folgenden Liste ausgewählt werden, ohne die Erfindung damit einzuschränken: Nucleotide (z.B. Adenosin-5'-monophosphat, Cytidin-5'-monophosphat, Inosin-5'-monophosphat) oder deren pharmazeutisch akzeptablen Salze, Lactisole, 2,4-Dihydroxybenzoesäure, 3-Hydroxybenzoesäure, Natriumsalze (z.B. Natriumchlorid, Natriumlactat, Natriumcitrat, Natriumacetat, Natriumgluconoat), Hydroxyflavanone (z.B. Eriodictyol, Homoeriodictyol oder deren Natriumsalze), insbesondere gemäß EP 1 258 200, Hydroxybenzoesäureamide (z.B. 2,4-Dihydroxybenzoesäurevanillylamid, 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxybenzoesäure-*N*-4-(hydroxy-3-methoxybenzyl)amid, 4-Hydroxybenzoesäure-*N*-(4-hydroxy-3-methoxybenzyl)amid, 2,4-Dihydroxybenzoesäure-*N*-(4-hydroxy-3-methoxybenzyl)amid-Mono-natriumsalz, 2,4-Dihydroxybenzoesäure-*N*-2-(4-hydroxy-3-methoxyphenyl)ethylamid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid (Aduncamid), 4-Hydroxybenzoesäurevanillylamid), Hydroxydeoxybenzoinen (z.B. 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon), Hesperetin nach WO 2007/014879, Diacetyltrimere nach WO 2006/058893, Deoxybenzoine nach WO 2006/106023, Gingerdione nach WO 2007/003527, Gemische zur Verstärkung des salzigen Geschmacks nach PCT/EP 2006/067120 und darauf basierenden Dokumenten, Aminosäuren (z.B. gamma-Aminobuttersäure, Taurin, beta-Alanin, Arginin, Ornithin, Lysin), Divanillin nach WO 2004/078302, Pellitorinen nach WO 2004/043960 und WO 2004/000787, Lactone wie oben erwähnt, dabei vorzugsweise Nonenolid und/oder 4-Methyl-5-hydroxy-hexansäurelacton oder deren Mischungen, oder Gemische von Molkeproteinen mit Lecithinen.

Besonders bevorzugte Geschmackskorrigenzien sind 2,4-Dihydroxybenzoesäure, 3-Hydroxybenzoesäure, Natriumlactat, Natriumcitrat, Natriumacetat, Natriumgluconoat, Hydroxyflavanone (z.B. Eriodictyol, Homoeriodictyol oder deren Natriumsalze), insbesondere gemäß EP 1 258 200, Hydroxybenzoesäureamide (z.B. 2,4-Dihydroxybenzoesäurevanillylamid, 2,4-*Dihydroxybenzoesäure-N-(4-hydroxy-3-methoxybenzyl)amid,* 2,4,6-Trihydroxybenzoesäure-*N*-(4-hydroxy-3-methoxybenzyl)amid, 2-Hydroxybenzoesäure-*N*-4-(hydroxy-3-methoxybenzyl)amid , 4-Hydroxybenzoesäure-*N*-(4-hydroxy-3-methoxybenzyl)amid, 2,4-Dihydroxybenzoesäure-*N*-(4-hydroxy-3-methoxybenzyl)amid-Mono-natriumsalz, 2,4-Dihydroxybenzoesäure-*N*-2-(4-hydroxy-3-methoxyphenyl)ethylamid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid (Aduncamid), 4-Hydroxybenzoesäurevanillylamid), Hydroxydeoxybenzoinen (z.B. 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon), Hesperetin nach WO 2007/014879, Diacetyltrimere nach WO 2006/058893, Deoxybenzoine nach WO 2006/106023, Gingerdione nach WO 2007/003527, Divanillin nach WO 2004/078302, Pellitorinen nach WO 2004/043960 und WO 2004/000787, Lactone wie oben erwähnt, dabei vorzugsweise Nonenolid und/oder 4-Methyl-5-hydroxy-hexansäurelacton oder deren Mischungen.

Insbesondere bevorzugt sind Hydroxyflavanone (z.B. Eriodictyol, Homoeriodictyol oder deren Natriumsalze), insbesondere gemäß EP 1 258 200, 2,4-Dihydroxybenzoesäurevanillylamid, 2,4,6-Trihydroxybenzoesäure-*N*-(4-hydroxy-3-meth oxybenzyl)amid, 2,4-Dihydroxybenzoesäure-*N*-(4-hydroxy-3-methoxybenzyl)amid-Mono-natriumsalz, Hesperetin nach WO 2007/014879, Diacetyltrimere nach WO 2006/058893, Gingerdion-[2] nach WO 2007/003527, Divanillin nach WO 2004/078302, trans-Pellitorin nach WO 2004/043960 und WO 2004/000787, Lactone wie oben erwähnt, dabei vorzugsweise Nonenolid und/oder 4-Methyl-5-hydroxy-hexansäurelacton oder deren Mischungen.

In einer weiteren, besonders bevorzugten Ausführung der Erfindung werden die erfindungsgemäßen 4-Hydroxychalkonderivate der Formel (I), deren Salze oder Gemische, gegebenenfalls zusammen mit den oben genannten weiteren Geschmackskorrigenzien, in Kombination mit einem oder mehreren Stoffen, die einen unangenehmen Nachgeschmack mit einem nicht unangenehmen Primärgeschmack (beispielsweise süß, salzig, würzig, sauer) haben, verwendet, wobei man erfindungsgemäße Zubereitungen erhält, die auch zur Herstellung verzehrsfertiger Zubereitungen dienen können, in der der unangenehme Nachgeschmack der unangenehm schmeckenden Stoffe dann maskiert oder vermindert wird. In diesen Zubereitungen werden als Stoffe, die einen unangenehmen Nachgeschmack mit einem nicht unangenehmen Primärgeschmack haben, vor allem Kaliumsalze (insbesondere Kaliumchlorid, Kaliumgluconat, Kaliumcarbonat, Kaliumsulfat, Kaliumlactat, Kaliumglutamat, Kaliumsuccinat oder Kaliummalat), Aspartam, Neotam, Superaspartam, Saccharin, Sucralose, Tagatose, Monellin, Stevioside, Rebaudioside, Hernandulcin, Thaumatin, Miraculin, Glycyrrhizin, Glycyrrhetinsäure oder deren Derivate, Cyclamat oder die pharmazeutisch akzeptablen Salze der vorgenannten Verbindungen, eingesetzt.

### Beispiele

Die Beispiele dienen zur Verdeutlichung der Erfindung, ohne den Schutzbereich der Patentansprüche einzuschränken.

### Beispiel 1: Phloretin, Verbindung 4

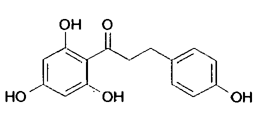

Es wurde kommerzielles Phloretin (Sigma-Aldrich, Best.-No. P 7912, CA-No. 60-82-2, Reinheit >98 %) verwendet.
Sensorisches Profil in 5 Gew.-%iger, wässriger Zuckerlösung (Sucrose):
süß, adstringierend, staubig-trocken

### Beispiel 2: Verbindung 6, 2',4,4'-Trihydroxy-3-methoxydihydrochalkon

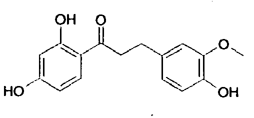

Die Synthese wurde in US 60/784,444, Beispiel 3 beschrieben.
Sensorisches Profil in 5 Gew.-%iger, wässriger Zuckerlösung (Sucrose):
relativ neutral, leichte Sirupnote

### Beispiel 3: Verbindung 10, 4,4'-Dihydroxy-3-methoxydihydrochalkon

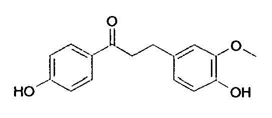

Die Synthese erfolgte analog zu der in US 60/784,444, Beispiel 3 beschrieben.
Sensorisches Profil in 5 Gew.-%iger, wässriger Zuckerlösung (Sucrose):
neutral

### Anwendungsbeispiel 1: Bitter-Reduzierung einer Bitterstofflösung

Um die Reduzierung des Bitter-Eindrucks zu quantifizieren, wurde die Bitterkeit einer wässrigen Bitterstofflösung und einer Probe, die die gleiche Menge Bitterstoff und eine wechselnde Menge der beispielhaften Verbindung enthielt, von einer Expertengruppe bestimmt (Einstufung 0 [nicht bitter] bis 10 [extrem bitter]). Die Auswertung erfolgte als Berechnung der Reduktion (in %) des Bittereindrucks aus den Durchschnittswerten der Einschätzungen der Bitterstofflösung bzw. der Bitterstoff und beispielhafte Verbindung enthaltenden Lösungen. 2,4-Dihydroxybenzoesäure (2,4-DHB) wurde als Vergleich aus US 5,643,941 verwendet.

**Tabelle: Bitterkeit einer Bitterstoff-Lösung und einer Bitterstoff und eine beispielhafte Verbindung enthaltenden Lösung (2,4-DHB = 2,4-Dihydroxybenzoesäure). "Prüfer positiv" bedeutet die Anzahl der Prüfer, die eine Maskierung feststellen konnten; als Fehler sind die 95 %-Konfidenzintervalle angegeben; p<x bedeutet die Signifikanz nach dem Student t-Test-Verfahren (vgl. Lehrbücher der Statistik), n.s. bedeutet nicht signifikant.**

| **Substanz** | **Bitterstoff** | **Prüfer** | | **Bitter-Eindruck (1-10)** | | **% Reduktion des Bitter-Eindrucks** |
|---|---|---|---|---|---|---|
| | | **gesamt** | **positiv** | **ohne** | **mit** | |
| 100 ppm 2,4-DHB | 500 ppm Coffein | 15 | 9 | 5,1 ±1,0 | 5,0 ± 1,0 | 3%, n.s. |
| 50 ppm Verbindung 4, Phloretin | 500 ppm Coffein | 16 | 13 | 5,5 ± 0,9 | 3,9 ± 1,8 | 28 %, p<0,05 |
| 30 ppm Verbindung 4, Phloretin | 5 ppm Chininhydrochlorid | 15 | 10 | 3,9 ±2,2 | 2,0 ± 1,4 | 25 %, n.s. |
| 30 ppm Verbindung 4, Phloretin | 250 ppm Salicin | 15 | 10 | 7,3±2,0 | 6,0 ± 1,8 | 18%,p<0,1 |
| Verbindung 6, 2',4,4'-Trihydroxy-3-methoxydihydrochalkon | 500 ppm Coffein | 15 | 11 | 4,4 ± 1,1 | 3,3 ± 0,7 | 24 %, n.s. |
| 50 ppm Verbindung 10, 4,4'-Dihydroxy-3-methoxydihydrochalkon | 500 ppm Coffein | 15 | 15 | 5,2 ± 0,9 | 3,7 ± 0,8 | 29 %, p < 0,05 |
| 50 ppm Verbindung 10, 4,4'-Dihydroxy-3-methoxydihydrochalkon | 5 ppm Chininhydrochlorid | 13 | 11 | 3,6 ± 1,0 | 2,4 ± 0,8 | 34 %, p < 0,1 |

### Anwendungsbeispiel 2: Reduktion der unangenehmen Geschmackseindrücke von Kaliumchlorid-haltigen Zubereitungen

Eine Fett- und Kochsalz-freie Fleischbrühe wird mit 0,4 Gew.-% Natriumchlorid und 0,4 Gew.-% Kaliumchlorid (jeweils auf das Gesamtgewicht bezogen) zubereitet (Vergleichslösung A). Die gleiche Lösung wird noch einmal zubereitet und ein Menge Phloretin (Verbindung 4 aus Beispiel 1) zugesetzt. Den Prüfern wird die Lösung A einmal als mit "Standardlösung" bezeichnet vorgesetzt, gleichzeitig in einer dem jeweiligen Prüfer unbekannten, zufälligen Reihenfolge die Lösung A und die Lösung B als "Testlösung 1" oder "Testlösung 2" (das heißt, Lösungen A bzw. B können für unterschiedliche Prüfer verschiedene Bezeichnungen haben; jeder Prüfer hat drei Lösungen zu testen). In einer ersten Testphase werden die Prüfer gefragt, ob eine der drei Lösungen anders als die beiden übrigen schmeckt (Unterschiedsprüfung im Dreieckstest). Wenn diese Frage mit "Nein" beantwortet wird, wird der Versuch für den Prüfer abgebrochen. Wenn die Frage mit "Ja" beantwortet wird, so muss der Prüfer die unten festgelegten Geschmacksqualitäten der von der "Standardlösung" abweichenden "Testlösung 1" oder "Testlösung 2" quantifizieren (relativ zur "Standardlösung", 0 = Geschmacksqualität identisch zum Standard, -4 sehr viel schwächer als Standard, +4 sehr viel stärker als Standard). Aus den Ergebnissen aller Prüfer wird der Durchschnitt aller Abweichungen gegen die "Standardlösung" für die einzelnen Geschmacksqualitäten berechnet.

| **Substanz** | **salzig** | **süß** | **sauer** | **bitter** | **Mundfülle** | **Würzigkeit** | **Anzahl Prüfer** | **Prüfer positiv** |
|---|---|---|---|---|---|---|---|---|
| Phloretin (Verbindung 4 aus Beispiel 1), 30 ppm | -0,7 | 0,9 | 0,1 | -0,4 | 0,3 | -0,1 | 21 | 16 (p < 0,05) |

Die beispielhafte Anwendung ist zwar etwas weniger salzig und süßer als die "Standardlösung", zeigt aber wesentlich weniger bitteren Nachgeschmack und wird daher präferiert.

### Anwendungsbeispiel 3: Sprühgetrocknete Zubereitung als Halbfertigware zur Aromatisierung von Fertigwaren

| **Inhaltsstoff** | **Einsatz in Gew.-%** |
|---|---|
| Trinkwasser | 60,8 % |
| Maltodextrin aus Weizen | 24,3 % |
| Gummi Arabicum | 6,1 % |
| Phloretin (Verbindung 4 aus Beispiel 1) | 8,8 % |

Das Trinkwasser wird in einem Behälter vorgelegt und das Maltodextrin und das Gummi Arabicum darin gelöst. Anschließend wird das Phloretin (Beispiel 1; Verbindung 4) mit einem Turrax in die Trägerstofflösung emulgiert. Die Temperatur der Sprühlösung sollte 30°C nicht überschreiten. Das Gemisch wird dann sprühgetrocknet (Solltemperatur Eingang: 185 - 195°C, Solltemperatur Ausgang: 70 - 75°C). Die sprühgetrocknete Halbfertigware enthält ca. 18 - 22 % des Wirkstoffs aus Beispiel 2.

Analog können sprühgetrocknete Zubereitungen als Halbfertigwaren aus Beispiel 2 (Verbindung 6) hergestellt werden.

### Anwendungsbeispiel 4: Schwarztee-Zubereitung

| **Inhaltsstoff** | **Einsatz in Gew.-%** |
|---|---|
| schwarzer Tee, Ceylon, Blattware | 94,00 % |
| Halbfertigware aus Anwendungsbeispiel 2, enthaltend ca. 18 - 22 % Phloretin (Verbindung 4 aus Beispiel 1) | 6 % |

Der Tee und die Halbfertigware werden gemischt und in Teebeutel aus Filterpapier abgepackt. Zur Anwendung wird ein Teebeutel in 100 - 250 ml kochendes Wasser aufgegossen und 2 - 5 min ziehen gelassen.

### Anwendungsbeispiel 5: Schwarztee-Zubereitung in Kombination mit Homoeriodictyol-Natriumsalz

| **Inhaltsstoff** | **Einsatz in Gew.-%** |
|---|---|
| schwarzer Tee, Ceylon, Blattware | 94,00 % |
| Halbfertigware aus Anwendungsbeispiel 2, enthaltend ca. 18 - 22 % Phloretin (Verbindung 4 aus Beispiel 1) | 3 % |
| Halbfertigware aus Homoeriodictyol-Natriumsalz gemäß EP 1258200 B1, sprühgetrocknet analog zu Anwendungsbeispiel 2 | 3 % |

Der Tee und die Halbfertigwaren werden gemischt und in Teebeutel aus Filterpapier abgepackt. Zur Anwendung wird ein Teebeutel in 100 - 250 ml kochendes Wasser aufgegossen und 2-5 min ziehen gelassen.

### Anwendungsbeispiel 6: Verwendung in einem Soja-Getränk

Phloretin aus Beispiel 1 wurde in Ethanol vorgelöst und zu einer Sojamilch aus einem lokalen Supermarkt hinzugefügt. Die Mischung wurde zusammen mit dem Milcharoma im Becherglas verrührt.

| **Inhaltsstoff** | **Einsatz in Gew.-%** |
|---|---|
| Sojamilch (lokaler Supermarkt) | 99,8 % |
| Milcharoma | 0,1 % |
| 10 % Phloretin (Verbindung 4 aus Beispiel 1) in Ethanol | 0,1 % |

### Anwendungsbeispiel 7: Verwendung in einem Soja-Getränk in Kombination mit γ-Aminobuttersäure

γ-Aminobuttersäure wurde in Wasser und Phloretin (Verbindung 4, Beispiel 1) Ethanol vorgelöst und zu einer Sojamilch aus einem lokalen Supermarkt hinzugefügt. Die Mischung wurde zusammen mit dem Milcharoma im Becherglas verrü h rt.

| **Inhaltsstoff** | **Einsatz in Gew.-%** |
|---|---|
| Sojamilch (lokaler Supermarkt) | 99,7 % |
| Milcharoma | 0,1 % |
| 10 % Phloretin (Verbindung 4 aus Beispiel 1) in Ethanol | 0,1 % |
| 1 % γ-Aminobuttersäure in Wasser | 0,1 % |

### Anwendungsbeispiel 8: Verwendung in einer bitteren Schokolade

Die sprühgetrocknete Zubereitung aus Anwendungsbeispiel 2 wurde in die bei 40°C geschmolzene Schokolade eingearbeitet und die flüssige Masse in Tafelform gegossen und nach dem dem Fachmann bekannten Temperungsverfahren abgekühlt, wobei man eine Essschokolade erhält.

| **Inhaltsstoff** | **Einsatz in Gew.-%** |
|---|---|
| Bitterschokolade, min. 85 % Kakao (Handelsprodukt) | 99,8 % |
| Sprühgetrocknete Zubereitung aus Anwendungsbeispiel 2 | 0,2 % |

Die so zubereitete Schokolade wurde von den trainierten Fachleuten als weniger bitter, weniger adstringierend und insgesamt als mehr abgerundet bezeichnet.

### Anwendungsbeispiel 9: Aroma zur Reduzierung der bitteren Geschmacksqualität von Kaliumsalz-haltiqen Zubereitungen

| **Inhaltsstoff** | **Einsatz in Gew.-%** | | | |
|---|---|---|---|---|
| | Zubereitung A | Zubereitung B | Zubereitung C | Zubereitung D |
| Phloretin (Verbindung 4 aus Beispiel 1) | 1 % | 1 % | 1 % | 1 % |
| Hesperetin, sprühgetrocknet | | 9 % | | |
| trans-Pellitorin nach WO 2004/043960, sprühgetrocknet | 16,5 % | 16,5 % | | |
| Divanillin, sprühgetrocknet | 5 % | 5 % | 5 % | 5 % |
| Maltodextrin | 68,5 % | 65,7% | 94,7 % | 93,7 % |
| Nonenolid | | | | 0,3 % |
| 4-Methyl-5-hydroxyhexansäurelacton | | | 0,3 % | |

### Anwendungsbeispiel 10: Maskierung von Kaliumsalz-haltigen Brotaufstrichen

In eine marktgängige Halbfettmargarine (40 % Fett) wurden in die wässrige Phase Kaliumchlorid eingebracht (4 %) (Standardzubereitung). Nach Aufteilung der Masse wurde in die eine Hälfte die Zubereitung D nach Anwendungsbeispiel 9 eingebracht und wieder homogenisiert (Testzubereitung). Die Standardzubereitung wurde gegen die Testzubereitung sensorisch verglichen: die Standardzubereitung ist deutlich salziger und bitterer, insbesondere im Nachgeschmack als die Testzubereitung. Die Testzubereitung wurde von den meisten Prüfern bevorzugt.

### Anwendungsbeispiel 11: Verbesserung von Grüntee-Zubereitungen

Zubereitung A: Vergleichszubereitung ohne Maskierung
Zubereitung B und C: erfindungsgemäße Zubereitungen

| **Inhaltsstoff** | **Einsatz in Gew.-%** | | |
|---|---|---|---|
| | Zubereitung A | Zubereitung B | Zubereitung C |
| Sucrose | 5% | 5% | 5% |
| Grünteeextrakt (wässrig, sprühgetr.) | 0,33 % | 0,33 % | 0,33 % |
| Phloretin (Verbindung 4 aus Beispiel 1) | - | 0,005 % (=50 ppm) | - |
| 4,4'-Dihydroxy-3-methoxydihydrochalkon (Verbindung 10 aus Beispiel 3) | - | - | 0,005 % (=50 ppm) |
| Wasser | auf 100 % auffüllen | auf 100 % auffüllen | auf 100 % auffüllen |

Die Zutaten wurden in der angegebenen Reihenfolge gemischt und anschließend von einem Panel (15 Prüfer) folgendermaßen bewertet: jeder Prüfer bekam 3 Proben, von denen eine die Probe A war (dem Prüfer kenntlich gemacht, Vergleichsprobe), eine weitere ebenfalls Probe A (dem Prüfer aber nicht bekannt) und die letzte Probe Probe B (dem Prüfer aber nicht bekannt) war. Die beiden lezteren Proben wurden in randomisierter Reihenfolge den Prüfern präsentiert. In der ersten Testphase mussten die Prüfer entscheiden, ob sie eine der beiden letzteren Proben von der Vergleichsprobe unterscheiden konnten. Wenn ja, sollten die Prüfer anhand einer Skala von -4 (viel schwächer),-3,-2,-1,0 (keine Unterscheidung), 1,2,3,4 (viel stärker) entscheiden, inwieweit sich Adstringens, Bitterkeit und Süße (jeweils für sich bewertet) von der bekannten Vergleichsporbe unterscheiden.

Der Versuch wurde identisch noch mal durchgeführt, nur das Zubereitung C statt B geprüft wurde.

In der folgenden Tabelle sind die Ergebnisse dargestellt:

| Zubereitung | Abweichung Bitterkeit gegen Zubereitung A | Abweichung Adstringens gegen Zubereitung A | Abweichung Süße gegen Zubereitung A | Signifikanter Unterschied (p<0,05)? |
|---|---|---|---|---|
| B | -0,20 | 0,27 | 0,53 | ja |
| C | -0,27 | 0,20 | -0,47 | ja |

In Zubereitung B war die Bitterkeit gegenüber der Vergleichszubereitung A reduziert, die Adstringens leicht erhöht, insbesondere auch die Süße verstärkt. In Zubereitung C war die Bitterkeit gegenüber der Vergleichszubereitung A deutlich reduziert, Adstringens wenig erhöht und die Süße etwas reduziert.

Weitere Anwendungsbeispiele sind in der US 60/784,444 beschrieben.

Weitere Ausführungsformen sind nachfolgend beschrieben.

### Ausführungsform 1

Verwendung eines 4-Hydroxychalkonderivats der Formel (I) 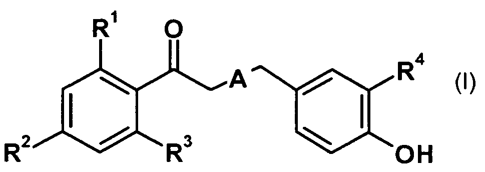 wobei
A eine Einfach- oder Doppelbindung darstellt,
R¹, R² und R³, unabhängig voneinander, jeweils H, OH oder (vorzugsweise C₁-C₄-) Alkoxy bedeuten können, mit der Maßgabe, dass mindestens einer der Reste R¹ bis R³ OH bedeutet,
und R⁴ H, OH oder (vorzugsweise C₁-C₄-) Alkoxy bedeutet,
und/oder
- eines Salzes eines solchen 4-Hydroxychalkonderivats der Formel (I),
- einer Mischung umfassend oder bestehend aus zwei oder mehr unterschiedlichen 4-Hydroxychalkonderivaten der Formel (I), wobei R¹, R², R³ und R⁴ jeweils die oben angegebene Bedeutung haben,
- einer Mischung umfassend oder bestehend aus Salzen von zwei oder mehr unterschiedlichen 4-Hydroxychalkonderivaten der Formel (I), wobei R¹, R², R³ und R⁴ jeweils die oben angegebene Bedeutung haben,
oder
- einer Mischung umfassend oder bestehend aus
   einem 4-Hydroxychalkonderivat der Formel (I) oder zwei oder mehr unterschiedlichen 4-Hydroxychalkonderivaten der Formel (I), wobei R¹, R², R³ und R⁴ jeweils die oben angegebene Bedeutung haben, und
   einem Salz eines 4-Hydroxychalkonderivats der Formel (I) oder zwei oder mehr Salzen von unterschiedlichen 4-Hydroxychalkonderivaten der Formel (I), wobei R¹, R², R³ und R⁴ jeweils die oben angegebene Bedeutung haben,
   zum Verändern, Maskieren oder Vermindern des unangenehmen Geschmackseindrucks eines unangenehm schmeckenden Stoffes.

### Ausführungsform 2

Verwendung nach Ausführungsform 1, wobei in Formel (I)
A eine Einfachbindung darstellt, und entweder R¹ OH bedeutet, und R² und R³, unabhängig voneinander, H oder OH bedeuten, oder R² OH bedeutet, und R¹ und R³ H bedeuten
und R⁴ ein H oder Methoxy bedeutet,
oder eines Salzes eines solchen 4-Hydroxychalkonderivats.

### Ausführungsform 3

Verwendung nach Ausführungsform 1, eines 4-Hydroxychalkonderivats der Formel (I) ausgewählt aus der Gruppe bestehend aus
3-(4-Hydroxyphenyl)-1-(2-hydroxyphenyl)propan-1-on (Verbindung 1)
3-(4-Hydroxyphenyl)-1-(2,4-dihydroxyphenyl)propan-1-on (Davidigenin, Verbindung 2)
3-(4-Hydroxyphenyl)-1-(2,6-dihydroxyphenyl)propan-1-on (Verbindung 3)
3-(4-Hydroxyphenyl)-1-(2,4,6-trihydroxyphenyl)propan-1-on (Phloretin, Verbindung 4)
3-(4-Hydroxy-3-methoxyphenyl)-1-(2-hydroxyphenyl)propan-1-on (Verbindung 5)
3-(4-Hydroxy-3-methoxyphenyl)-1-(2,4-dihydroxyphenyl)propan-1-on (Verbindung 6)
3-(4-Hydroxy-3-methoxyphenyl)-1-(2,6-dihydroxyphenyl)propan-1-on (Verbindung 7)
3-(4-Hydroxy-3-methoxyphenyl)-1-(2,4,6-trihydroxyphenyl)propan-1-on (Verbindung 8)
3-(3,4-Dihydroxyphenyl)-1-(2,4,6-trihydroxyphenyl)propan-1-on (3-Hydroxyphloretin, Verbindung 9),
3-(4-Hydroxy-3-methoxyphenyl)-1-(4-hydroxyphenyl)propan-1-on (Verbindung 10)
- einem Salz eines solchen 4-Hydroxychalkonderivats der Formel (I),
- einer Mischung umfassend oder bestehend aus zwei oder mehr aus der besagten Gruppe ausgewählten 4-Hydroxychalkonderivaten der Formel (I),
- einer Mischung umfassend oder bestehend aus Salzen von zwei oder mehr aus der besagten Gruppe ausgewählten 4-Hydroxychalkonderivaten der Formel (I)
oder
- einer Mischung umfassend oder bestehend aus
   einem aus der besagten Gruppe ausgewählten 4-Hydroxychalkonderivat der Formel (I) oder zwei oder mehr aus der besagten Gruppe ausgewählten 4-Hydroxychalkonderivaten der Formel (I) und
   einem Salz eines aus der besagten Gruppe ausgewählten 4-Hydroxychalkonderivats der Formel (I) oder zwei oder mehr Salzen von unterschiedlichen aus der besagten Gruppe ausgewählten 4-Hydroxychalkonderivaten der Formel (I).

### Ausführungsform 4

Verwendung nach einer der vorherigen Ausführungsformen, wobei eine, mehrere oder sämtliche Hydroxy-Gruppen der Verbindung der Formel (I) deprotoniert sind und eine entsprechende Menge von Gegenkationen vorliegt, und wobei die Gegenkationen ausgewählt sind aus der Gruppe bestehend aus einfach positiv geladenen Kationen der ersten Haupt- und Nebengruppe, Ammoniumionen, Trialkylammoniumionen, zweifach positiv geladene Kationen der zweiten Haupt- und Nebengruppe sowie dreifach positiv geladene Kationen der dritten Haupt- und Nebengruppe, und deren Mischungen.

### Ausführungsform 5

Verwendung nach einer der vorangehenden Ausführungsformen, zusammen mit zumindest einem Lacton, zum Verändern, Maskieren oder Vermindern des unangenehmen Geschmackseindrucks eines unangenehm schmeckenden Stoffes.

### Ausführungsform 6

Verwendung nach Ausführungsform 5, wobei zusätzlich Nonenolid und/oder 4-Methyl-5-hydroxyhexansäurelacton verwendet wird.

### Ausführungsform 7

Verwendung nach einer der Ausführungsformen 5 bis 6 zum Verändern, Maskieren oder Vermindern des insbesondere bitteren Geschmacks eines Kaliumsalzes, vorzugsweise von Kaliumchlorid.

### Ausführungsform 8

Verwendung nach einer der vorangehenden Ausführungsformen in einer zur oralen Aufnahme bestimmten pharmazeutischen oder einer der Ernährung, der Mundpflege oder dem Genuss dienenden Zubereitung.

### Ausführungsform 9

Verwendung nach einer der vorherigen Ausführungsformen, wobei zusätzlich Eriodictyol, Homoerdiodictyol und/oder ein Natriumsalz derselben verwendet wird.

### Ausführungsform 10

Zubereitung, umfassend
a)
   - ein 4-Hydroxychalkonderivat der Formel (I) 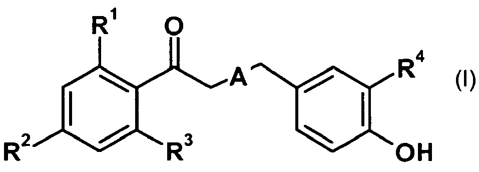 wobei
      - A: eine Einfach- oder Doppelbindung darstellen kann,
      - R¹, R² und R³,: unabhängig voneinander, jeweils H, OH oder (vorzugsweise C₁-C₄-) Alkoxy bedeuten können, mit der Maßgabe, dass mindestens einer der Reste R¹ bis R³ OH bedeutet,
      - und R⁴: H, OH oder (vorzugsweise C₁-C₄-) Alkoxy bedeutet,
   und/oder
   - ein Salz eines solchen 4-Hydroxychalkonderivats der Formel (I),
   - eine Mischung umfassend oder bestehend aus zwei oder mehr unterschiedlichen 4-Hydroxychalkonderivaten der Formel (I), wobei R¹, R², R³ und R⁴ jeweils die oben angegebene Bedeutung haben,
   - eine Mischung umfassend oder bestehend aus Salzen von zwei oder mehr unterschiedlichen 4-Hydroxychalkonderivaten der Formel (I), wobei R¹, R², R³ und R⁴ jeweils die oben angegebene Bedeutung haben, oder
   - eine Mischung umfassend oder bestehend aus
      einem 4-Hydroxychalkonderivat der Formel (I) oder zwei oder mehr unterschiedlichen 4-Hydroxychalkonderivaten der Formel (I), wobei R¹, R², R³ und R⁴ jeweils die oben angegebene Bedeutung haben, und
      einem Salz eines 4-Hydroxychalkonderivats der Formel (I) oder zwei oder mehr Salzen von unterschiedlichen 4-Hydroxychalkonderivaten der Formel (I), wobei R¹, R², R³ und R⁴ jeweils die oben angegebene Bedeutung haben,
   und
b) ein oder mehrere unangenehm schmeckenden Stoffe, mit der Maßgabe, dass
   i) das 4-Hydroxychalkonderivat der Formel (I) nicht Phloretin ist, wenn die Zubereitung Hesperetin, Phloridzin oder ein anderes Phloretingylycosid enthält, und/oder dass
   ii) wenn die Zubereitung eine oder mehrere süß schmeckende Stoffe enthält, die Süße der Zubereitung maximal 1 % Sucrose-Äquivalente beträgt.

### Ausführungsform 11

Zubereitung nach Ausführungsform 10, wobei in Formel (I)
A eine Einfachbindung darstellt, und entweder R¹ OH bedeutet, und R² und R³, unabhängig voneinander, H oder OH bedeuten oder R² OH bedeutet, und R¹ und R³ H bedeuten
und R⁴ ein H oder Methoxy bedeutet, oder ein Salz eines solchen 4-Hydroxychalkonderivats.

### Ausführungsform 12

Zubereitung nach Ausführungsform 10 oder 11, umfassend
- ein 4-Hydroxychalkonderivat der Formel (I) ausgewählt aus der Gruppe bestehend aus
   3-(4-Hydroxyphenyl)-1-(2-hydroxyphenyl)propan-1-on (Verbindung 1)
   3-(4-Hydroxyphenyl)-1-(2,4-dihydroxyphenyl)propan-1-on (Davidigenin, Verbindung 2)
   3-(4-Hydroxyphenyl)-1-(2,6-dihydroxyphenyl)propan-1-on (Verbindung 3)
   3-(4-Hydroxyphenyl)-1-(2,4,6-trihydroxyphenyl)propan-1-on (Phloretin, Verbindung 4)
   3-(4-Hydroxy-3-methoxyphenyl)-1-(2-hydroxyphenyl)propan-1-on (Verbindung 5)
   3-(4-Hydroxy-3-methoxyphenyl)-1-(2,4-dihydroxyphenyl)propan-1-on (Verbindung 6)
   3-(4-Hydroxy-3-methoxyphenyl)-1-(2,6-dihydroxyphenyl)propan-1-on (Verbindung 7)
   3-(4-Hydroxy-3-methoxyphenyl)-1-(2,4,6-trihydroxyphenyl)propan-1-on (Verbindung 8)
   3-(3,4-Dihydroxyphenyl)-1-(2,4,6-trihydroxyphenyl)propan-1-on (3-Hydroxyphloretin, Verbindung 9),
   3-(4-Hydroxy-3-methoxyphenyl)-1-(4-hydroxyphenyl)propan-1-on (Verbindung 10)
- ein Salzes eines solchen 4-Hydroxychalkonderivats der Formel (I),
- eine Mischung umfassend oder bestehend aus zwei oder mehr aus der besagten Gruppe ausgewählten 4-Hydroxychalkonderivaten der Formel (I),
- eine Mischung umfassend oder bestehend aus Salzen von zwei oder mehr aus der besagten Gruppe ausgewählten 4-Hydroxychalkonderivaten der Formel (I)
oder
- eine Mischung umfassend oder bestehend aus
   einem aus der besagten Gruppe ausgewählten 4-Hydroxychalkonderivat der Formel (I) oder zwei oder mehr aus der besagten Gruppe ausgewählten 4-Hydroxychalkonderivaten der Formel (I) und
   einem Salz eines aus der besagten Gruppe ausgewählten 4-Hydroxychalkonderivats der Formel (I) oder zwei oder mehr Salzen von unterschiedlichen aus der besagten Gruppe ausgewählten 4-Hydroxychalkonderivaten der Formel (I).

### Ausführungsform 13

Zubereitung nach einer der Ausführungsformen 10, 11 oder 12, dadurch gekennzeichnet, dass der oder die unangenehm schmeckenden Stoffe ausgewählt ist bzw. sind aus der Gruppe bestehend aus: Xanthinalkaloide; Xanthine, insbesondere Coffein, Theobromin, Theophyllin und Methylxanthine; Alkaloide, insbesondere Chinin, Brucin, Strychnin, Nicotin; phenolische Glycoside, insbesondere Salicin, Arbutin; Flavonoidglycoside, insbesondere Neohespereidin, Hesperidin, Naringin, Quercitrin, Rutin; bitter schmeckende Chalcone oder Chalconglycoside, insbesondere Phloridzin, hydrolisierbare Tannine, Gallus- oder Elagsäureester von Kohlenhydraten, insbesondere Pentagalloylglucose; nichthydrolisierbare Tannine, insbesondere galloylierte Catechine oder Epicatechine und deren Oligomeren, insbesondere Proanthyocyanidine oder Procyanidine, Thearubigenin; Flavone, insbesondere Quercetin, Taxifolin, Myricetin; Phenole, insbesondere Salicin; Polyphenole, insbesondere γ-Oryzanol, Kaffeesäure oder deren Ester; terpenoide Bitterstoffe, insbesondere Limonoide wie Limonin oder Nomilin aus Zitrusfrüchten, Lupolone und Humolone aus Hopfen, Iridoide, Secoiridoide; Absinthin; Amarogentin; metallische Salze, insbesondere Kalium-, Magnesium- und Calciumsalze, Kaliumchlorid, Kaliumgluconat, Kaliumcarbonat, Kaliumsulfat, Kaliumlactat, Kaliumglutamat, Kaliumsuccinat, Kaliummalat, Natriumsulfat, Magnesiumsulfat; pharmazeutische Wirkstoffe, insbesondere Fluorchinolon-Antibiotika, Paracetamol, Aspirin, β-Lactam-Antibiotika, Ambroxol, Propylthiouracil [PROP], Guaifenesin; Vitamine, insbesondere Vitamin H, Vitamine aus der B-Reihe wie Vitamin B1, B2, B6, B12, Niacin, Panthotensäure); Denatoniumbenzoat; Sucraloseoctaacetat; Eisensalze; Aluminiumsalze; Zinksalze; Harnstoff; ungesättigte Fettsäuren, insbesondere ungesättigte Fettsäuren in Emulsionen; bitter schmeckende Aminosäuren, insbesondere Leucin, Isoleucin, Valin, Tryptophan, Prolin, Histidin, Tyrosin, Lysin oder Phenylalanin; und bitter schmeckende Peptide, insbesondere Peptide mit einer Aminosäure aus der Gruppe Leucin, Isoleucin, Valin, Tryptophan, Prolin oder Phenylalanin am N- oder C-Terminus.

### Ausführungsform 14

Zubereitung nach einer der Ausführungsformen 10 bis 13, ferner umfassend ein oder mehrere Lactone, vorzugsweise Nonenolid und/oder 4-Methyl-5-hydroxyhexansäurelacton.

### Ausführungsform 15

Zubereitung nach einer der Ausführungsformen 10 bis 14, wobei die Zubereitung eine orale pharmazeutische Zubereitung ist, eine der Ernährung dienende Zubereitung ist, eine dem Genuss dienende Zubereitung ist oder eine der Mundhygiene dienende Zubereitung ist.

### Ausführungsform 16

Zubereitung nach einer der Ausführungsformen 10 bis 15, ferner umfassend zumindest einen unangenehm schmeckenden Stoff, wobei die Menge des unangenehm schmeckenden Stoffes ausreicht, um in einer Vergleichszubereitung, die kein 4-Hydroxychalkonderivat der Formel (I), Salz oder Gemisch eines solchen 4-Hydroxychalkonderivats umfasst, aber ansonsten identisch zusammengesetzt ist, als unangenehmer Geschmack wahrgenommen zu werden, und die Menge des 4-Hydroxychalkonderivats der Formel (I), Salz oder des Gemisch eines solchen 4-Hydroxychalkonderivats in der Zubereitung ausreicht, um im Vergleich mit der Vergleichszubereitung den unangenehmen Geschmackseindruck des unangenehm schmeckenden Stoffes sensorisch zu verändern, zu maskieren oder zu vermindern.

### Ausführungsform 17

Zubereitung nach einer der Ausführungsformen 10 bis 16, dadurch gekennzeichnet, dass die Zubereitung, bezogen auf ihr Gesamtgewicht, insgesamt 0,000001 bis 95 Gew.-% an 4-Hydroxychalkonderivat(en) und, soweit vorhanden, seinen/ihren Salzen enthält.

### Ausführungsform 18

Zubereitung nach einer der Ausführungsformen 10 bis 17, dadurch gekennzeichnet, dass sie als Halbfertigware, als Riech-, Aroma- oder Geschmacksstoffkomposition oder als Würzmischung vorliegt.

### Ausführungsform 19

Zubereitung nach einer der Ausführungsformen 10 bis 18, ferner umfassend zumindest eine weitere Substanz zum Verändern, Maskieren oder Vermindern des unangenehmen Geschmackseindrucks eines unangenehm schmeckenden Stoffes.

### Ausführungsform 20

Verfahren zum Verändern, Maskieren oder Vermindern des unangenehmen Geschmackseindrucks eines unangenehm schmeckenden Stoffes, umfassend den Schritt des Vermischens eines unangenehm schmeckenden Stoffes mit
- einem 4-Hydroxychalkonderivat der Formel (I) 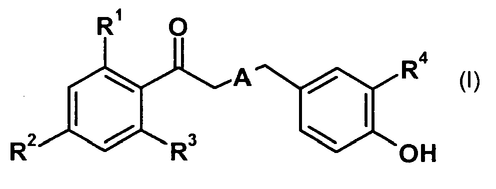 wobei
   - A: eine Einfach- oder Doppelbindung darstellen kann,
   - R¹, R² und R³,: unabhängig voneinander, jeweils H, OH oder (vorzugsweise C₁-C₄-) Alkoxy bedeuten können, mit der Maßgabe, dass mindestens einer der Reste R¹ bis R³ OH bedeutet,
   - und R⁴: H, OH oder (vorzugsweise C₁-C₄-) Alkoxy bedeutet,
und/oder
- einem Salz eines solchen 4-Hydroxychalkonderivats der Formel (I),
- einer Mischung umfassend oder bestehend aus zwei oder mehr unterschiedlichen 4-Hydroxychalkonderivaten der Formel (I), wobei R¹, R², R³ und R⁴ jeweils die oben angegebene Bedeutung haben,
- einer Mischung umfassend oder bestehend aus Salzen von zwei oder mehr unterschiedlichen 4-Hydroxychalkonderivaten der Formel (I), wobei R¹, R², R³ und R⁴ jeweils die oben angegebene Bedeutung haben,
oder
- einer Mischung umfassend oder bestehend aus
   einem 4-Hydroxychalkonderivat der Formel (I) oder zwei oder mehr unterschiedlichen 4-Hydroxychalkonderivaten der Formel (I), wobei R¹, R², R³ und R⁴ jeweils die oben angegebene Bedeutung haben, und
   einem Salz eines 4-Hydroxychalkonderivats der Formel (I) oder zwei oder mehr Salzen von unterschiedlichen 4-Hydroxychalkonderivaten der Formel (I), wobei R¹, R², R³ und R⁴ jeweils die oben angegebene Bedeutung haben.

### Ausführungsform 21

Verfahren nach Ausführungsform 20, wobei in Formel (I)
A eine Einfachbindung darstellt, und entweder R¹ OH bedeutet, und R² und R³, unabhängig voneinander, H oder OH bedeuten oder R² OH bedeutet, und R¹ und R³ H bedeuten
und R⁴ ein H oder Methoxy bedeutet,
oder das 4-Hydroxychalkonderivat in Form eines Salzes vorliegt.

### Ausführungsform 22

Verfahren nach Ausführungsform 20 oder 21, wobei
- das 4-Hydroxychalkonderivat der Formel (I) ausgewählt ist aus der Gruppe bestehend aus
   3-(4-Hydroxyphenyl)-1-(2-hydroxyphenyl)propan-1-on (Verbindung 1)
   3-(4-Hydroxyphenyl)-1-(2,4-dihydroxyphenyl)propan-1-on (Davidigenin, Verbindung 2)
   3-(4-Hydroxyphenyl)-1-(2,6-dihydroxyphenyl)propan-1-on (Verbindung 3)
   3-(4-Hydroxyphenyl)-1-(2,4,6-trihydroxyphenyl)propan-1-on (Phloretin, Verbindung 4)
   3-(4-Hydroxy-3-methoxyphenyl)-1-(2-hydroxyphenyl)propan-1-on (Verbindung 5)
   3-(4-Hydroxy-3-methoxyphenyl)-1-(2,4-dihydroxyphenyl)propan-1-on (Verbindung 6)
   3-(4-Hydroxy-3-methoxyphenyl)-1-(2,6-dihydroxyphenyl)propan-1-on (Verbindung 7)
   3-(4-Hydroxy-3-methoxyphenyl)-1-(2,4,6-trihydroxyphenyl)propan-1-on (Verbindung 8)
   3-(3,4-Dihydroxyphenyl)-1-(2,4,6-trihydroxyphenyl)propan-1-on (3-Hydroxyphloretin, Verbindung 9),
   3-(4-Hydroxy-3-methoxyphenyl)-1-(4-hydroxyphenyl)propan-1-on (Verbindung 10)
- das Salz ein Salzes eines solchen 4-Hydroxychalkonderivats der Formel (I) ist,
- die Mischung eine Mischung umfassend oder bestehend aus zwei oder mehr aus der besagten Gruppe ausgewählten 4-Hydroxychalkonderivaten der Formel (I) ist,
- die Mischung eine Mischung umfassend oder bestehend aus Salzen von zwei oder mehr aus der besagten Gruppe ausgewählten 4-Hydroxychalkonderivaten der Formel (I) ist,
oder
- die Mischung eine Mischung umfassend oder bestehend aus
   einem aus der besagten Gruppe ausgewählten 4-Hydroxychalkonderivat der Formel (I) oder zwei oder mehr aus der besagten Gruppe ausgewählten 4-Hydroxychalkonderivaten der Formel (I) und
   einem Salz eines aus der besagten Gruppe ausgewählten 4-Hydroxychalkonderivats der Formel (I) oder zwei oder mehr Salzen von unterschiedlichen aus der besagten Gruppe ausgewählten 4-Hydroxychalkonderivaten der Formel (I) ist.

### Ausführungsform 23

Verfahren nach einer der Ausführungsformen 20, 21 oder 22, wobei eine, mehrere oder sämtliche Hydroxy-Gruppen des 4-Hydroxychalkonderivats der Formel (I) deprotoniert sind und eine entsprechende Menge von Gegenkationen vorliegt, und wobei die Gegenkationen ausgewählt sind aus der Gruppe bestehend aus einfach positiv geladenen Kationen der ersten Haupt- und Nebengruppe, Ammoniumionen, Trialkylammoniumionen, zweifach positiv geladene Kationen der zweiten Haupt- und Nebengruppe sowie dreifach positiv geladene Kationen der dritten Haupt- und Nebengruppe, und deren Mischungen.

### Ausführungsform 24

Verfahren nach einer der Ausführungsformen 20, 21, 22 oder 23, zusätzlich umfassend das Vermischen des unangenehm schmeckenden Stoffes mit zumindest einem Lacton, vorzugsweise von Nonenolid und/oder 4-Methyl-5-hydroxyhexansäurelacton.

### Ausführungsform 25

Verfahren nach einer der Ausführungsformen 20, 21, 22, 23 oder 24, wobei der unangenehm schmeckende Stoff ein bitter schmeckender Stoff ist, vorzugsweise ein Kaliumsalz, und besonders bevorzugt Kaliumchlorid.

### Ausführungsform 26

Verfahren nach einer der Ausführungsformen 20, 21, 22, 23, 24 oder 25, zusätzlich umfassend das Vermischen des unangenehm schmeckenden Stoffes mit Eriodictyol, Homoerdiodictyol und/oder einem Natriumsalz derselben.

## Patentansprüche

1. Verwendung
- eines 4-Hydroxychalkonderivats der Formel (I) 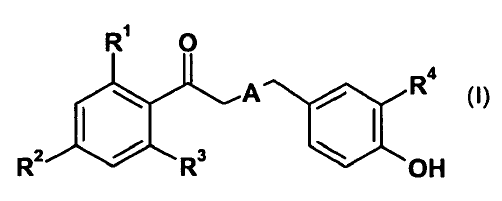 wobei
A eine Einfach- oder Doppelbindung darstellt,
R¹, R² und R³, unabhängig voneinander, jeweils H, OH oder (vorzugsweise C₁-C₄-) Alkoxy bedeuten können, mit der Maßgabe, dass mindestens einer der Reste R¹ bis R³ OH bedeutet,
und R⁴ H, OH oder (vorzugsweise C₁-C₄-) Alkoxy bedeutet,
und/oder
- eines Salzes eines solchen 4-Hydroxychalkonderivats der Formel (I),
- einer Mischung umfassend oder bestehend aus zwei oder mehr unterschiedlichen 4-Hydroxychalkonderivaten der Formel (I), wobei R¹, R², R³ und R⁴ jeweils die oben angegebene Bedeutung haben,
- einer Mischung umfassend oder bestehend aus Salzen von zwei oder mehr unterschiedlichen 4-Hydroxychalkonderivaten der Formel (I), wobei R¹, R², R³ und R⁴ jeweils die oben angegebene Bedeutung haben,
oder
- einer Mischung umfassend oder bestehend aus
einem 4-Hydroxychalkonderivat der Formel (I) oder zwei oder mehr unterschiedlichen 4-Hydroxychalkonderivaten der Formel (I), wobei R¹, R², R³ und R⁴ jeweils die oben angegebene Bedeutung haben, und
einem Salz eines 4-Hydroxychalkonderivats der Formel (I) oder zwei oder mehr Salzen von unterschiedlichen 4-Hydroxychalkonderivaten der Formel (I), wobei R¹, R², R³ und R⁴ jeweils die oben angegebene Bedeutung haben,
zum Maskieren oder Vermindern des unangenehmen Geschmackseindrucks eines unangenehm schmeckenden Stoffes.

2. Verwendung nach Anspruch 1,
- eines 4-Hydroxychalkonderivats der Formel (I) ausgewählt aus der Gruppe bestehend aus
3-(4-Hydroxyphenyl)-1-(2-hydroxyphenyl)propan-1-on (Verbindung 1)
3-(4-Hydroxyphenyl)-1-(2,4-dihydroxyphenyl)propan-1-on (Davidigenin, Verbindung 2)
3-(4-Hydroxyphenyl)-1-(2,6-dihydroxyphenyl)propan-1-on (Verbindung 3)
3-(4-Hydroxyphenyl)-1-(2,4,6-trihydroxyphenyl)propan-1-on (Phloretin, Verbindung 4)
3-(4-Hydroxy-3-methoxyphenyl)-1-(2-hydroxyphenyl)propan-1-on (Verbindung 5)
3-(4-Hydroxy-3-methoxyphenyl)-1-(2,4-dihydroxyphenyl)propan-1-on (Verbindung 6)
3-(4-Hydroxy-3-methoxyphenyl)-1-(2,6-dihydroxyphenyl)propan-1-on (Verbindung 7)
3-(4-Hydroxy-3-methoxyphenyl)-1-(2,4,6-trihydroxyphenyl)propan-1-on (Verbindung 8)
3-(3,4-Dihydroxyphenyl)-1-(2,4,6-trihydroxyphenyl)propan-1-on (3-Hydroxyphloretin, Verbindung 9),
3-(4-Hydroxy-3-methoxyphenyl)-1-(4-hydroxyphenyl)propan-1-on (Verbindung 10)
- einem Salz eines solchen 4-Hydroxychalkonderivats der Formel (I),
- einer Mischung umfassend oder bestehend aus zwei oder mehr aus der besagten Gruppe ausgewählten 4-Hydroxychalkonderivaten der Formel (I),
- einer Mischung umfassend oder bestehend aus Salzen von zwei oder mehr aus der besagten Gruppe ausgewählten 4-Hydroxychalkonderivaten der Formel (I)
oder
- einer Mischung umfassend oder bestehend aus
einem aus der besagten Gruppe ausgewählten 4-Hydroxychalkonderivat der Formel (I) oder zwei oder mehr aus der besagten Gruppe ausgewählten 4-Hydroxychalkonderivaten der Formel (I) und
einem Salz eines aus der besagten Gruppe ausgewählten 4-Hydroxychalkonderivats der Formel (I) oder zwei oder mehr Salzen von unterschiedlichen aus der besagten Gruppe ausgewählten 4-Hydroxychalkonderivaten der Formel (I).

3. Verwendung nach einem der vorangehenden Ansprüche, zusammen mit zumindest einem Lacton und vorzugsweise zusammen mit Nonenolid und/oder 4-Methyl-5-hydroxyhexansäurelacton, zum Maskieren oder Vermindern des unangenehmen Geschmackseindrucks eines unangenehm schmeckenden Stoffes, vorzugsweise des bitteren Geschmacks eines Kaliumsalzes und besonders bevorzugt von Kaliumchlorid.

4. Zubereitung, umfassend
a)
- ein 4-Hydroxychalkonderivat der Formel (I) 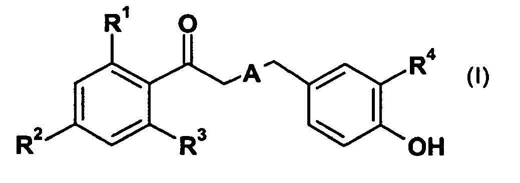 wobei
A eine Einfach- oder Doppelbindung darstellt,
R¹, R² und R³, unabhängig voneinander, jeweils H, OH oder (vorzugsweise C₁-C₄-) Alkoxy bedeuten können, mit der Maßgabe, dass mindestens einer der Reste R¹ bis R³ OH bedeutet,
und R⁴ H, OH oder (vorzugsweise C₁-C₄-) Alkoxy bedeutet,
und/oder
- ein Salz eines solchen 4-Hydroxychalkonderivats der Formel (I),
- eine Mischung umfassend oder bestehend aus zwei oder mehr unterschiedlichen 4-Hydroxychalkonderivaten der Formel (I), wobei R¹, R², R³ und R⁴ jeweils die oben angegebene Bedeutung haben,
- eine Mischung umfassend oder bestehend aus Salzen von zwei oder mehr unterschiedlichen 4-Hydroxychalkonderivaten der Formel (I), wobei R¹, R², R³ und R⁴ jeweils die oben angegebene Bedeutung haben,
oder
- eine Mischung umfassend oder bestehend aus
einem 4-Hydroxychalkonderivat der Formel (I) oder zwei oder mehr unterschiedlichen 4-Hydroxychalkonderivaten der Formel (I), wobei R¹, R², R³ und R⁴ jeweils die oben angegebene Bedeutung haben, und
einem Salz eines 4-Hydroxychalkonderivats der Formel (I) oder zwei oder mehr Salzen von unterschiedlichen 4-Hydroxychalkonderivaten der Formel (I), wobei R¹, R², R³ und R⁴ jeweils die oben angegebene Bedeutung haben,
und
b) ein oder mehrere unangenehm schmeckenden Stoffe, mit der Maßgabe, dass
i) das 4-Hydroxychalkonderivat der Formel (I) nicht Phloretin ist, wenn die Zubereitung Hesperetin, Phloridzin oder ein anderes Phloretingylycosid enthält, und dass
ii) wenn die Zubereitung eine oder mehrere süß schmeckende Stoffe enthält, die Süße der Zubereitung maximal 1 % Sucrose-Äquivalente beträgt.

5. Zubereitung nach Anspruch 4, umfassend
- ein 4-Hydroxychalkonderivat der Formel (I) ausgewählt aus der Gruppe bestehend aus
3-(4-Hydroxyphenyl)-1-(2-hydroxyphenyl)propan-1-on (Verbindung 1)
3-(4-Hydroxyphenyl)-1-(2,4-dihydroxyphenyl)propan-1-on (Davidigenin, Verbindung 2)
3-(4-Hydroxyphenyl)-1-(2,6-dihydroxyphenyl)propan-1-on (Verbindung 3)
3-(4-Hydroxyphenyl)-1-(2,4,6-trihydroxyphenyl)propan-1-on (Phloretin, Verbindung 4)
3-(4-Hydroxy-3-methoxyphenyl)-1-(2-hydroxyphenyl)propan-1-on (Verbindung 5)
3-(4-Hydroxy-3-methoxyphenyl)-1-(2,4-dihydroxyphenyl)propan-1-on (Verbindung 6)
3-(4-Hydroxy-3-methoxyphenyl)-1-(2,6-dihydroxyphenyl)propan-1-on (Verbindung 7)
3-(4-Hydroxy-3-methoxyphenyl)-1-(2,4,6-trihydroxyphenyl)propan-1-on (Verbindung 8)
3-(3,4-Dihydroxyphenyl)-1-(2,4,6-trihydroxyphenyl)propan-1-on (3-Hydroxyphloretin, Verbindung 9),
3-(4-Hydroxy-3-methoxyphenyl)-1-(4-hydroxyphenyl)propan-1-on (Verbindung 10)
- ein Salzes eines solchen 4-Hydroxychalkonderivats der Formel (I),
- eine Mischung umfassend oder bestehend aus zwei oder mehr aus der besagten Gruppe ausgewählten 4-Hydroxychalkonderivaten der Formel (I),
- eine Mischung umfassend oder bestehend aus Salzen von zwei oder mehr aus der besagten Gruppe ausgewählten 4-Hydroxychalkonderivaten der Formel (I)
oder
- eine Mischung umfassend oder bestehend aus
einem aus der besagten Gruppe ausgewählten 4-Hydroxychalkonderivat der Formel (I) oder zwei oder mehr aus der besagten Gruppe ausgewählten 4-Hydroxychalkonderivaten der Formel (I) und
einem Salz eines aus der besagten Gruppe ausgewählten 4-Hydroxychalkonderivats der Formel (I) oder zwei oder mehr Salzen von unterschiedlichen aus der besagten Gruppe ausgewählten 4-Hydroxychalkonderivaten der Formel (I).

6. Zubereitung nach einem der Ansprüche 4 oder 5, **dadurch gekennzeichnet, dass** der oder die unangenehm schmeckenden Stoffe ausgewählt ist bzw. sind aus der Gruppe bestehend aus: Xanthinalkaloide; Xanthine, insbesondere Coffein, Theobromin, Theophyllin und Methylxanthine; Alkaloide, insbesondere Chinin, Brucin, Strychnin, Nicotin; phenolische Glycoside, insbesondere Salicin, Arbutin; Flavonoidglycoside, insbesondere Neohespereidin, Hesperidin, Naringin, Quercitrin, Rutin; bitter schmeckende Chalcone oder Chalconglycoside, insbesondere Phloridzin, hydrolisierbare Tannine, Gallus- oder Elagsäureester von Kohlenhydraten, insbesondere Pentagalloylglucose; nichthydrolisierbare Tannine, insbesondere galloylierte Catechine oder Epicatechine und deren Oligomeren, insbesondere Proanthyocyanidine oder Procyanidine, Thearubigenin; Flavone, insbesondere Quercetin, Taxifolin, Myricetin; Phenole, insbesondere Salicin; Polyphenole, insbesondere γ-Oryzanol, Kaffeesäure oder deren Ester, terpenoide Bitterstoffe, insbesondere Limonoide wie Limonin oder Nomilin aus Zitrusfrüchten, Lupolone und Humolone aus Hopfen, Iridoide, Secoiridoide; Absinthin; Amarogentin; metallische Salze, insbesondere Kalium-, Magnesium- und Calciumsalze, Kaliumchlorid, Kaliumgluconat, Kaliumcarbonat, Kaliumsulfat, Kaliumlactat, Kaliumglutamat, Kaliumsuccinat, Kaliummalat, Natriumsulfat, Magnesiumsulfat; pharmazeutische Wirkstoffe, insbesondere Fluorchinolon-Antibiotika, Paracetamol, Aspirin, β-Lactam-Antibiotika, Ambroxol, Propylthiouracil [PROP], Guaifenesin; Vitamine, insbesondere Vitamin H, Vitamine aus der B-Reihe wie Vitamin B1, B2, B6, B12, Niacin, Panthotensäure); Denatoniumbenzoat; Sucraloseoctaacetat; Eisensalze; Aluminiumsalze; Zinksalze; Harnstoff; ungesättigte Fettsäuren, insbesondere ungesättigte Fettsäuren in Emulsionen; bitter schmeckende Aminosäuren, insbesondere Leucin, Isoleucin, Valin, Tryptophan, Prolin, Histidin, Tyrosin, Lysin oder Phenylalanin; und bitter schmeckende Peptide, insbesondere Peptide mit einer Aminosäure aus der Gruppe Leucin, Isoleucin, Valin, Tryptophan, Prolin oder Phenylalanin am N- oder C-Terminus.

7. Zubereitung nach einem der Ansprüche 4 bis 6, ferner umfassend ein oder mehrere Lactone, vorzugsweise Nonenolid und/oder 4-Methyl-5-hydroxyhexansäurelacton.

8. Zubereitung nach einem der Ansprüche 4 bis 7, wobei die Zubereitung eine orale pharmazeutische Zubereitung ist, eine der Ernährung dienende Zubereitung ist, eine dem Genuss dienende Zubereitung ist oder eine der Mundhygiene dienende Zubereitung ist.

9. Zubereitung nach einem der Ansprüche 4 bis 8, ferner umfassend zumindest einen unangenehm schmeckenden Stoff, wobei die Menge des unangenehm schmeckenden Stoffes ausreicht, um in einer Vergleichszubereitung, die kein 4-Hydroxychalkonderivat der Formel (I), Salz oder Gemisch eines solchen 4-Hydroxychalkonderivats umfasst, aber ansonsten identisch zusammengesetzt ist, als unangenehmer Geschmack wahrgenommen zu werden, und die Menge des 4-Hydroxychalkonderivats der Formel (I), Salz oder des Gemisch eines solchen 4-Hydroxychalkonderivats in der Zubereitung ausreicht, um im Vergleich mit der Vergleichszubereitung den unangenehmen Geschmackseindruck des unangenehm schmeckenden Stoffes sensorisch zu verändern, zu maskieren oder zu vermindern.

10. Zubereitung nach einem der Ansprüche 4 bis 9, **dadurch gekennzeichnet, dass** die Zubereitung, bezogen auf ihr Gesamtgewicht, insgesamt 0,000001 bis 95 Gew.-% an 4-Hydroxychalkonderivat(en) und, soweit vorhanden, seinen/ihren Salzen enthält.

11. Zubereitung nach einem der Ansprüche 4 bis 10, **dadurch gekennzeichnet, dass** sie als Halbfertigware, als Riech-, Aroma- oder Geschmacksstoffkomposition oder als Würzmischung vorliegt.

12. Zubereitung nach einem der Ansprüche 4 bis 11, ferner umfassend zumindest eine weitere Substanz zum Maskieren oder Vermindern des unangenehmen Geschmackseindrucks eines unangenehm schmeckenden Stoffes.

13. Verfahren zum Maskieren oder Vermindern des unangenehmen Geschmackseindrucks eines unangenehm schmeckenden Stoffes, umfassend den Schritt des Vermischens eines unangenehm schmeckenden Stoffes mit
- einem 4-Hydroxychalkonderivat der Formel (I) 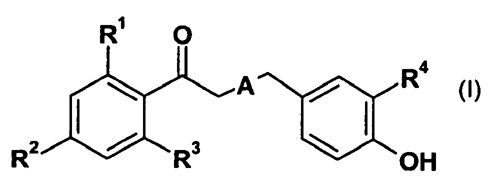 wobei
A eine Einfach- oder Doppelbindung darstellen kann,
R¹, R² und R³, unabhängig voneinander, jeweils H, OH oder (vorzugsweise C₁-C₄-) Alkoxy bedeuten können, mit der Maßgabe, dass mindestens einer der Reste R¹ bis R³ OH bedeutet,
und R⁴ H, OH oder (vorzugsweise C₁-C₄-) Alkoxy bedeutet,
und/oder
- einem Salz eines solchen 4-Hydroxychalkonderivats der Formel (I),
- einer Mischung umfassend oder bestehend aus zwei oder mehr unterschiedlichen 4-Hydroxychalkonderivaten der Formel (I), wobei R¹, R², R³ und R⁴ jeweils die oben angegebene Bedeutung haben,
- einer Mischung umfassend oder bestehend aus Salzen von zwei oder mehr unterschiedlichen 4-Hydroxychalkonderivaten der Formel (I), wobei R¹, R², R³ und R⁴ jeweils die oben angegebene Bedeutung haben,
oder
- einer Mischung umfassend oder bestehend aus
einem 4-Hydroxychalkonderivat der Formel (I) oder zwei oder mehr unterschiedlichen 4-Hydroxychalkonderivaten der Formel (I), wobei R¹, R², R³ und R⁴ jeweils die oben angegebene Bedeutung haben, und
einem Salz eines 4-Hydroxychalkonderivats der Formel (I) oder zwei oder mehr Salzen von unterschiedlichen 4-Hydroxychalkonderivaten der Formel (I), wobei R¹, R², R³ und R⁴ jeweils die oben angegebene Bedeutung haben.

14. Verfahren nach Anspruch 13, wobei
- das 4-Hydroxychalkonderivat der Formel (I) ausgewählt ist aus der Gruppe bestehend aus
3-(4-Hydroxyphenyl)-1-(2-hydroxyphenyl)propan-1-on (Verbindung 1)
3-(4-Hydroxyphenyl)-1-(2,4-dihydroxyphenyl)propan-1-on (Davidigenin, Verbindung 2)
3-(4-Hydroxyphenyl)-1-(2,6-dihydroxyphenyl)propan-1-on (Verbindung 3)
3-(4-Hydroxyphenyl)-1-(2,4,6-trihydroxyphenyl)propan-1-on (Phloretin, Verbindung 4)
3-(4-Hydroxy-3-methoxyphenyl)-1-(2-hydroxyphenyl)propan-1-on (Verbindung 5)
3-(4-Hydroxy-3-methoxyphenyl)-1-(2,4-dihydroxyphenyl)propan-1-on (Verbindung 6)
3-(4-Hydroxy-3-methoxyphenyl)-1-(2,6-dihydroxyphenyl)propan-1-on (Verbindung 7)
3-(4-Hydroxy-3-methoxyphenyl)-1-(2,4,6-trihydroxyphenyl)propan-1-on (Verbindung 8)
3-(3,4-Dihydroxyphenyl)-1-(2,4,6-trihydroxyphenyl)propan-1-on (3-Hydroxyphloretin, Verbindung 9),
3-(4-Hydroxy-3-methoxyphenyl)-1-(4-hydroxyphenyl)propan-1-on (Verbindung 10)
- das Salz ein Salzes eines solchen 4-Hydroxychalkonderivats der Formel (I) ist,
- die Mischung eine Mischung umfassend oder bestehend aus zwei oder mehr aus der besagten Gruppe ausgewählten 4-Hydroxychalkonderivaten der Formel (I) ist,
- die Mischung eine Mischung umfassend oder bestehend aus Salzen von zwei oder mehr aus der besagten Gruppe ausgewählten 4-Hydroxychalkonderivaten der Formel (I) ist,
oder
- die Mischung eine Mischung umfassend oder bestehend aus einem aus der besagten Gruppe ausgewählten 4-Hydroxychalkonderivat der Formel (I) oder zwei oder mehr aus der besagten Gruppe ausgewählten 4-Hydroxychalkonderivaten der Formel (I) und
einem Salz eines aus der besagten Gruppe ausgewählten 4-Hydroxychalkonderivats der Formel (I) oder zwei oder mehr Salzen von unterschiedlichen aus der besagten Gruppe ausgewählten 4-Hydroxychalkonderivaten der Formel (I) ist.

## Claims

1. Use
- of a 4-hydroxychalcone derivative of the formula (I) 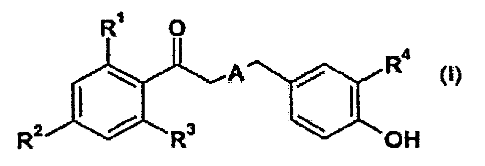 wherein
A represents a single or double bond,
R¹, R² and R³ independently of each other can each denote H, OH or (preferably C₁-C₄-)alkoxy, with the proviso that at least one of the radicals R¹ to R³ denotes OH,
and R⁴ denotes H, OH or (preferably C₁-C₄-)alkoxy,
and/or
- of a salt of such a 4-hydroxychalcone derivative of the formula (I),
- of a mixture comprising or consisting of two or more different 4-hydroxychalcone derivatives of the formula (I), wherein R¹, R², R³ and R⁴ each have the abovementioned meaning,
- of a mixture comprising or consisting of salts of two or more different 4-hydroxychalcone derivatives of the formula (I), wherein R¹, R², R³ and R⁴ each have the abovementioned meaning,
or
- of a mixture comprising or consisting of a 4-hydroxychalcone derivative of the formula (I) or two or more different 4-hydroxychalcone derivatives of the formula (I) , wherein R¹, R², R³ and R⁴ each have the abovementioned meaning, and
a salt of a 4-hydroxychalcone derivative of the formula (I) or two or more salts of different 4-hydroxychalcone derivatives of the formula (I), wherein R¹, R², R³ and R⁴ each have the abovementioned meaning,
for masking or reducing the unpleasant taste impression of an unpleasantly tasting substance.

2. Use according to claim 1,
- of a 4-hydroxychalcone derivative of the formula (I) chosen from the group consisting of
3-(4-hydroxyphenyl)-1-(2-hydroxyphenyl)propan-1-one (compound 1)
3-(4-hydroxyphenyl)-1-(2,4-dihydroxyphenyl)propan-1-one (davidigenin, compound 2)
3-(4-hydroxyphenyl)-1-(2,6-dihydroxyphenyl)propan-1-one (compound 3)
3-(4-hydroxyphenyl)-1-(2,4,6-trihydroxyphenyl)propan-1-one (phloretin, compound 4)
3-(4-hydroxy-3-methoxyphenyl)-1-(2-hydroxyphenyl)propan-1-one (compound 5)
3-(4-hydroxy-3-methoxyphenyl)-1-(2,4-dihydroxyphenyl)propan-1-one (compound 6)
3-(4-hydroxy-3-methoxyphenyl)-1-(2,6-dihydroxyphenyl)propan-1-one (compound 7)
3-(4-hydroxy-3-methoxyphenyl)-1-(2,4,6-trihydroxyphenyl)propan-1-one (compound 8)
3-(3,4-dihydroxyphenyl)-1-(2,4,6-trihydroxyphenyl)propan-1-one (3-hydroxyphloretin, compound 9),
3-(4-hydroxy-3-methoxyphenyl)-1-(4-hydroxyphenyl)propan-1-one (compound 10)
- a salt of such a 4-hydroxychalcone derivative of the formula (I),
- a mixture comprising or consisting of two or more 4-hydroxychalcone derivatives of the formula (I) chosen from the said group,
- a mixture comprising or consisting of salts of two or more 4-hydroxychalcone derivatives of the formula (I) chosen from the said group
or
- a mixture comprising or consisting of a 4-hydroxychalcone derivative of the formula (I) chosen from the said group or two or more 4-hydroxychalcone derivatives of the formula (I) chosen from the said group and
a salt of a 4-hydroxychalcone derivative of the formula (I) chosen from the said group or two or more salts of different 4-hydroxychalcone derivatives of the formula (I) chosen from the said group.

3. Use according to one of the preceding claims, together with at least one lactone and preferably together with nonenolide and/or 4-methyl-5-hydroxyhexanoic acid lactone, for masking or reducing the unpleasant taste impression of an unpleasantly tasting substance, preferably of the bitter taste of a potassium salt and particularly preferably of potassium chloride.

4. Formulation comprising
a)
- a 4-hydroxychalcone derivative of the formula (I) 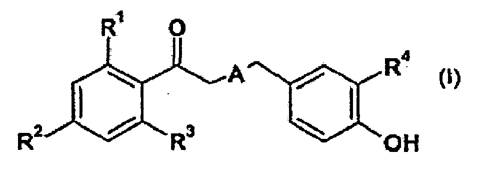 wherein
A represents a single or double bond,
R¹, R² and R³ independently of each other can each denote H,
OH or (preferably C₁-C₄-)alkoxy, with the proviso that at least one of the radicals R¹ to R³ denotes OH,
and R⁴ denotes H, OH or (preferably C₁-C₄-)alkoxy,
and/or
- a salt of such a 4-hydroxychalcone derivative of the formula (I),
- a mixture comprising or consisting of two or more different 4-hydroxychalcone derivatives of the formula (I), wherein R¹, R², R³ and R⁴ each have the abovementioned meaning,
- a mixture comprising or consisting of salts of two or more different 4-hydroxychalcone derivatives of the formula (I), wherein R¹, R², R³ and R⁴ each have the abovementioned meaning,
or
- a mixture comprising or consisting of
a 4-hydroxychalcone derivative of the formula (I) or two or more different 4-hydroxychalcone derivatives of the formula (I), wherein R¹, R², R³ and R⁴ each have the abovementioned meaning, and
a salt of a 4-hydroxychalcone derivative of the formula (I) or two or more salts of different 4-hydroxychalcone derivatives of the formula (I), wherein R¹, R², R³ and R⁴ each have the abovementioned meaning,
and
b) one or more unpleasantly tasting substances, with the proviso that
i) the 4-hydroxychalcone derivative of the formula (I) is not phloretin if the formulation contains hesperitin, phloridzin or another phloretin glycoside, and that
ii) if the formulation contains one or more sweet-tasting substances, the sweetness of the formulation is a maximum of 1 % sucrose equivalents.

5. Formulation according to claim 4, comprising
- a 4-hydroxychalcone derivative of the formula (I) chosen from the group consisting of
3-(4-hydroxyphenyl)-1-(2-hydroxyphenyl)propan-1-one (compound 1)
3-(4-hydroxyphenyl)-1-(2,4-dihydroxyphenyl)propan-1-one (davidigenin, compound 2)
3-(4-hydroxyphenyl)-1-(2,6-dihydroxyphenyl)propan-1-one (compound 3)
3-(4-hydroxyphenyl)-1-(2,4,6-trihydroxyphenyl)propan-1-one (phloretin, compound 4)
3-(4-hydroxy-3-methoxyphenyl)-1-(2-hydroxyphenyl)propan-1-one (compound 5)
3-(4-hydroxy-3-methoxyphenyl)-1-(2,4-dihydroxyphenyl)propan-1-one (compound 6)
3-(4-hydroxy-3-methoxyphenyl)-1-(2,6-dihydroxyphenyl)propan-1-one (compound 7)
3-(4-hydroxy-3-methoxyphenyl)-1-(2,4,6-trihydroxyphenyl)propan-1-one (compound 8)
3-(3,4-dihydroxyphenyl)-1-(2,4,6-trihydroxyphenyl)propan-1-one (3-hydroxyphloretin, compound 9),
3-(4-hydroxy-3-methoxyphenyl)-1-(4-hydroxyphenyl)propan-1-one (compound 10)
- a salt of such a 4-hydroxychalcone derivative of the formula (I),
- a mixture comprising or consisting of two or more 4-hydroxychalcone derivatives of the formula (I) chosen from the said group,
- a mixture comprising.or consisting of salts of two or more 4-hydroxychalcone derivatives of the formula (I) chosen from the said group
or
- a mixture comprising or consisting of
a 4-hydroxychalcone derivative of the formula (I) chosen from the said group or two or more 4-hydroxychalcone derivatives of the formula (I) chosen from the said group and
a salt of a 4-hydroxychalcone derivative of the formula (I) chosen from the said group or two or more salts of different 4-hydroxychalcone derivatives of the formula (I) chosen from the said group.

6. Formulation according to one of claims 4 or 5, **characterized in that** the unpleasantly tasting substance or substances is or are chosen from the group consisting of: xanthine alkaloids, xanthines, in particular caffeine, theobromine, theophylline and methylxanthine; alkaloids, in particular quinine, brucine, strychnine, nicotine; phenolic glycosides, in particular salicin, arbutin; flavonoid glycosides, in particular neohesperidin, hesperidin, naringin, quercitrin, rutin; bitter-tasting chalcones or chalcone glycosides, in particular phloridzin, hydrolysable tannins, gallic or ellagic acid esters of carbohydrates, in particular pentagalloylglucose; non-hydrolysable tannins, in particular galloylated catechols or epicatechols and oligomers thereof, in particular proanthyocyanidines or procyanidines, thearubigin; flavones, in particular quercertin, taxifolin, myricetin; phenols, in particular salicin; polyphenols, in particular γ-oryzanol, caffeic acid or esters thereof; terpenoid bitter substances, in particular limonoids such as limonin or nomilin from citrus fruits, lupolones and humulones from hops, iridoids, secoiridoids; absinthin; amarogentin; metal salts, in particular potassium, magnesium and calcium salts, potassium chloride, potassium gluconate, potassium carbonate, potassium sulfate, potassium lactate, potassium glutamate, potassium succinate, potassium malate, sodium sulfate, magnesium sulfate; pharmaceutical active compounds, in particular fluoroquinolone antibiotics, paracetamol, aspirin, β-lactam antibiotics, ambroxol, propylthiouracil [PROP], guaifenesin; vitamins, in particular vitamin H, vitamins from the B series, such as vitamin B1, B2, B6, B12, niacin, pantothenic acid; denatonium benzoate; sucralose octaacetate; iron salts; aluminium salts; zinc salts; urea; unsaturated fatty acids, in particular unsaturated fatty acids in emulsions; bitter-tasting amino acids, in particular leucine, isoleucine, valine, tryptophan, proline, histidine, tyrosine, lysine or phenylalanine; and bitter-tasting peptides, in particular peptides with an amino acid from the group of leucine, isoleucine, valine, tryptophan, proline or phenylalanine at the N- or C-terminus.

7. Formulation according to one of claims 4 to 6, furthermore comprising one or more lactones, preferably nonenolide and/or 4-methyl-5-hydroxyhexanoic acid lactone.

8. Formulation according to one of claims 4 to 7, wherein the formulation is an oral pharmaceutical formulation, a formulation serving for nutrition, a formulation serving for pleasure or a formulation serving for oral hygiene.

9. Formulation according to one of claims 4 to 8, furthermore comprising at least one unpleasantly tasting substance, wherein the amount of the unpleasantly tasting substance is sufficient to be perceived as an unpleasant taste in a comparison formulation which comprises no 4-hydroxychalcone derivative of the formula (I), salt or mixture of such a 4-hydroxychalcone derivative but is otherwise of identical composition, and the amount of the 4-hydroxychalcone derivative of the formula (I), salt or mixture of such a 4-hydroxychalcone derivative in the formulation is sufficient to sensorially modify, mask or reduce the unpleasant taste impression of the unpleasantly tasting substance compared with the comparison formulation.

10. Formulation according to one of claims 4 to 9, **characterized in that** the formulation contains, based on its total weight, a total of 0.000001 to 95 wt.% of 4-hydroxychalcone derivative(s) and, if present, its/their salts.

11. Formulation according to one of claims 4 to 10, **characterized in that** it is present as a semi-finished product, as an odoriferous, aroma or flavouring composition or as a seasoning mixture.

12. Formulation according to one of claims 4 to 11, furthermore comprising at least one further substance for masking or reducing the unpleasant taste impression of an unpleasantly tasting substance.

13. Method of masking or reducing the unpleasant taste impression of an unpleasantly tasting substance, comprising the step of mixing an unpleasantly tasting substance with
- a 4-hydroxychalcone derivative of the formula (I) 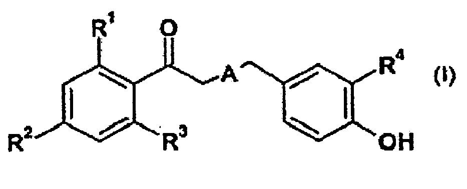 wherein
A represents a single or double bond,
R¹, R² and R³ independently of each other can each denote H, OH or (preferably C₁-C₄-)alkoxy, with the proviso that at least one of the radicals R¹ to R³ denotes OH,
and R⁴ denotes H, OH or (preferably C₁-C₄-)alkoxy,
and/or
- a salt of such a 4-hydroxychalcone derivative of the formula (I),
- a mixture comprising or consisting of two or more different 4-hydroxychalcone derivatives of the formula (I), wherein R¹, R², R³ and R⁴ each have the abovementioned meaning,
- a mixture comprising or consisting of salts of two or more different 4-hydroxychalcone derivatives of the formula (I), wherein R¹, R², R³ and R⁴ each have the abovementioned meaning,
or
- a mixture comprising or consisting of
a 4-hydroxychalcone derivative of the formula (I) or two or more different 4-hydroxychalcone derivatives of the formula (I), wherein R¹, R², R³ and R⁴ each have the abovementioned meaning, and
a salt of a 4-hydroxychalcone derivative of the formula (I) or two or more salts of different 4-hydroxychalcone derivatives of the formula (I), wherein R¹, R², R³ and R⁴ each have the abovementioned meaning.

14. Method according to claim 13, wherein
- the 4-hydroxychalcone derivative of the formula (I) is chosen from the group consisting of
3-(4-hydroxyphenyl)-1-(2-hydroxyphenyl)propan-1-one (compound 1)
3-(4-hydroxyphenyl)-1-(2,4-dihydroxyphenyl)propan-1-one (davidigenin, compound 2)
3-(4-hydroxyphenyl)-1-(2,6-dihydroxyphenyl)propan-1-one (compound 3)
3-(4-hydroxyphenyl)-1-(2,4,6-trihydroxyphenyl)propan-l-one (phloretin, compound 4)
3-(4-hydroxy-3-methoxyphenyl)-1-(2-hydroxyphenyl)propan-1-one (compound 5)
3-(4-hydroxy-3-methoxyphenyl)-1-(2,4-dihydroxyphenyl)propan-1-one (compound 6)
3-(4-hydroxy-3-methoxyphenyl)-1-(2,6-dihydroxyphenyl)propan-1-one (compound 7)
3-(4-hydroxy-3-methoxyphenyl)-1-(2,4,6-trihydroxyphenyl)propan-1-one (compound 8)
3-(3,4-dihydroxyphenyl)-1-(2,4,6-trihydroxyphenyl)propan-1-one (3-hydroxyphloretin, compound 9),
3-(4-hydroxy-3-methoxyphenyl)-1-(4-hydroxyphenyl)propan-1-one (compound 10)
- the salt is a salt of such a 4-hydroxychalcone derivative of the formula (I),
- the mixture is a mixture comprising or consisting of two or more 4-hydroxychalcone derivatives of the formula (I) chosen from the said group,
- the mixture is a mixture comprising or consisting of salts of two or more 4-hydroxychalcone derivatives of the formula (I) chosen from the said group
or
- the mixture is a mixture comprising or consisting of a 4-hydroxychalcone derivative of the formula (I) chosen from the said group or two or more 4-hydroxychalcone derivatives of the formula (I) chosen from the said group and
a salt of a 4-hydroxychalcone derivative of the formula (I) chosen from the said group or two or more salts of different 4-hydroxychalcone derivatives of the formula (I) chosen from the said group.

## Revendications

1. Utilisation
- d'un dérivé de la 4-hydroxychalcone de formule (I) 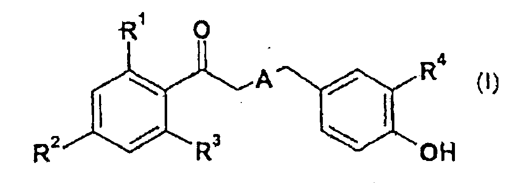 dans laquelle
A est une simple liaison ou une double liaison,
R¹, R² et R³ peuvent représenter chacun indépendamment des autres H, OH ou un groupe alcoxy de préférence en C₁-C₄, à la condition qu'au moins l'un des radicaux R¹ à
R³ soit OH,
et R⁴ est H, OH ou un groupe alcoxy de préférence en C₁-C₄,
et/ou
- d'un sel d'un tel dérivé de la 4-hydroxychalcone de formule (I),
- d'un mélange comprenant deux dérivés différents, ou plus, de la 4-hydroxychalcone de formule (I), chacun des radicaux R¹, R², R³ et R⁴ ayant les significations données ci-dessus, ou en étant constitué,
- d'un mélange comprenant des sels de deux dérivés différents, ou plus, de la 4-hydroxychalcone de formule (I), chacun des radicaux R¹, R², R³ et R⁴ ayant les significations données ci-dessus, ou en étant constitué,
ou
- d'un mélange comprenant un dérivé de la 4-hydroxychalcone de formule (I) ou deux dérivés différents, ou plus, de la 4-hydroxychalcone de formule (I), chacun des radicaux R¹, R², R³ et R⁴ ayant les significations données ci-dessus, et d'un sel d'un dérivé de la 4-hydroxychalcone de formule (I) ou de deux sels ou plus de dérivés différents de la 4-hydroxychalcone de formule (I), chacun des radicaux R¹, R², R³ et R⁴ ayant les significations données ci-dessus, ou en étant constitué,
pour masquer ou atténuer l'impression de goût désagréable d'une substance ayant un goût désagréable.

2. Utilisation selon la revendication 1,
- d'un dérivé de la 4-hydroxychalcone de formule (I) choisi dans le groupe consistant en :
la 3-(4-hydroxyphényl)-1-(2-hydroxyphényl)propan-1-one (composé 1) la 3-(4-hydroxyphényl)-1-(2,4-dihydroxyphényl)propan-1-one (davidigénine, composé 2)
la 3-(4-hydroxyphényl)-1-(2,6-dihydroxyphényl)propan-1-one (composé 3)
la 3-(4-hydroxyphényl)-1-(2,4,6-trihydroxyphényl)propan-1-one (florétine, composé 4)
la 3-(4-hydroxy-3-méthoxyphényl)-1-(2-hydroxyphényl)propan-1-one (composé 5)
la 3-(4-hydroxy-3-méthoxyphényl)-1-(2,4-dihydroxyphényl)propan-1-one (composé 6)
la 3-(4-hydroxy-3-méthoxyphényl)-1-(2,6-dihydroxyphényl)propan-1-one (composé 7)
la 3-(4-hydroxy-3-méthoxyphényl)-1-(2,4,6-trihydroxyphényl)propan-1-one (composé 8)
la 3-(3,4-dihydroxyphényl)-1-(2,4,6-trihydroxyphényl)propan-1-one (3-hydroxyflorétine, composé 9)
la 3-(4-hydroxy-3-méthoxyphényl)-1-(4-hydroxyphényl)propan-1-one (composé 10),
- un sel d'un tel dérivé de la 4-hydroxychalcone de formule (I),
- un mélange de deux dérivés de la 4-hydroxychalcone de formule (I) ou plus, choisis dans ledit groupe, ou en étant constitué,
- un mélange comprenant des sels de deux dérivés de la 4-hydroxychalcone de formule (I), ou plus, choisis dans ledit groupe, ou en étant constitué,
ou
- un mélange comprenant un dérivé de la 4-hydroxychalcone de formule (I) choisi dans ledit groupe, ou deux dérivés de la 4-hydroxychalcone de formule (I), ou plus, choisis dans ledit groupe, et un sel d'un dérivé de la 4-hydroxychalcone de formule (I), choisi dans ledit groupe, ou deux sels, ou plus, de dérivés différents de la 4-hydroxychalcone de formule (I), choisis dans ledit groupe, ou en étant constitué.

3. Utilisation selon l'une des revendications précédentes, avec au moins une lactone et de préférence avec un nonénolide et/ou de la 4-méthyl-5-hydroxyhexanolactone, pour masquer ou atténuer l'impression de goût désagréable d'une substance ayant un goût désagréable, de préférence le goût amer d'un sel de potassium et d'une manière particulièrement préférée du chlorure de potassium.

4. Préparation, comprenant :
a) un dérivé de la 4-hydroxychalcone de formule (I) 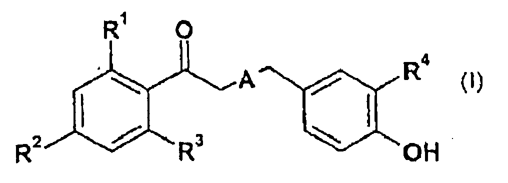 dans laquelle
A est une simple liaison ou une double liaison,
R¹, R² et R³ peuvent représenter chacun indépendamment des autres H, OH ou un groupe alcoxy de préférence en C₁-C₄, à la condition qu'au moins l'un des radicaux R¹ à R³ soit OH,
et R⁴ est H, OH ou un groupe alcoxy de préférence en C₁-C₄,
et/ou
- un sel d'un tel dérivé de la 4-hydroxychalcone de formule (I),
- un mélange comprenant deux dérivés différents, ou plus, de la 4-hydroxychalcone de formule (I), chacun des radicaux R¹, R², R³ et R⁴ ayant les significations données ci-dessus, ou en étant constitué,
- un mélange comprenant des sels de deux dérivés différents, ou plus, de la 4-hydroxychalcone de formule (I), chacun des radicaux R¹, R², R³ et R⁴ ayant les significations données ci-dessus, ou en étant constitué,
ou
- un mélange comprenant un dérivé de la 4-hydroxychalcone de formule (I) ou deux dérivés différents, ou plus, de la 4-hydroxychalcone de formule (I), chacun des radicaux R¹, R², R³ et R⁴ ayant les significations données ci-dessus, et un sel d'un dérivé de la 4-hydroxychalcone de formule (I), ou deux sels ou plus de dérivés différents de la 4-hydroxychalcone de formule (I), chacun des radicaux R¹, R², R³ et R⁴ ayant les significations données ci-dessus, ou en étant constitué,
et
b) une ou plusieurs substances ayant un goût désagréable, à la condition que
i) le dérivé de la 4-hydroxychalcone de formule (I) ne soit pas la florétine quand la préparation contient de l'hespérétine, de la floridzine ou un autre florétineglycoside, et que
ii) quand la préparation contient une ou plusieurs substances ayant un goût sucré, la sucrosité de la préparation soit d'au plus 1 % d'équivalent saccharose.

5. Procédé selon la revendication 4, comprenant :
- un dérivé de la 4-hydroxychalcone de formule (I), choisi dans le groupe consistant en :
la 3-(4-hydroxyphényl)-1-(2-hydroxyphényl)propan-1-one (composé 1)
la 3-(4-hydroxyphényl)-1-(2,4-dihydroxyphényl)propan-1-one (davidigénine, composé 2)
la 3-(4-hydroxyphényl)-1-(2,6-dihydroxyphényl)propan-1-one (composé 3)
la 3-(4-hydroxyphényl)-1-(2,4,6-trihydroxyphényl)propan-1-one (florétine, composé 4)
la 3-(4-hydroxy-3-méthoxyphényl)-1-(2-hydroxyphényl)propan-1-one (composé 5)
la 3-(4-hydroxy-3-méthoxyphényl)-1-(2,4-dihydroxyphényl)propan-1-one (composé 6)
la 3-(4-hydroxy-3-méthoxyphényl)-1-(2,6-dihydroxyphényl)propan-1-one (composé 7)
la 3-(4-hydroxy-3-méthoxyphényl)-1-(2,4,6-trihydroxyphényl)propan-1-one (composé 8)
la 3-(3,4-dihydroxyphényl)-1-(2,4,6-trihydroxyphényl)propan-1-one (3-hydroxyflorétine, composé 9)
la 3-(4-hydroxy-3-méthoxyphényl)-1-(4-hydroxyphényl)propan-1-one (composé 10),
- un sel d'un tel dérivé de la 4-hydroxychalcone de formule (I),
- un mélange comprenant deux dérivés de la 4-hydroxychalcone de formule (I), ou plus, choisis dans ledit groupe, ou en étant constitué,
- un mélange comprenant des sels de deux dérivés de la 4-hydroxychalcone de formule (I), ou plus, choisis dans ledit groupe, ou en étant constitué,
ou
- un mélange comprenant un dérivé de la 4-hydroxychalcone de formule (I), choisi dans ledit groupe, ou deux dérivés de la 4-hydroxychalcone de formule (I), ou plus, choisis dans ledit groupe, et un sel d'un dérivé de la 4-hydroxychalcone de formule (I) choisi dans ledit groupe ou deux sels ou plus de dérivés différents de la 4-hydroxychalcone de formule (I), choisis dans ledit groupe, ou en étant constitué.

6. Préparation selon l'une des revendications 4 ou 5, **caractérisée en ce que** la ou les substances ayant un goût désagréable sont choisies dans le groupe consistant en les xanthine-alcaloïdes ; les xanthines, en particulier la caféine, la théobromine, la théophylline et la méthylxanthine ; les alcaloïdes, en particulier la quinine, la brucine, la strychnine, la nicotine ; les glycosides phénoliques, en particulier la salicine, l'arbutine ; les flavanoïdeglycosides, en particulier la néohespéréidine, l'hespéridine, la naringine, la quercitrine, la rutine ; les chalcones ou chalconeglycosides ayant un goût amer, en particulier la floridzine, les tanins hydrolysables, les esters de l'acide gallique ou de l'acide élagique d'hydrates de carbone, en particulier le pentagalloylglucose ; les tanins non hydrolysables, en particulier les catéchines ou les épicatéchines galloylées et leurs oligomères, en particulier les proanthyocyanidines ou les procyanidines, la théarubigénine ; les flavones, en particulier la quercétine, la taxifoline, la myricétine ; les phénols, en particulier la salicine ; les polyphénols, en particulier le γ-oryzanol, l'acide caféique ou ses esters ; les substances amères terpénoïdes, en particulier les limonoïdes tels que la limonine ou la nomiline provenant des agrumes, les lupolones et les humolones provenant du houblon, les iridoïdes, les sécoiridoïdes ; l'absinthine ; l'amarogentine ; les sels métalliques, en particulier les sels de potassium, de magnésium et de calcium, le chlorure de potassium, le gluconate de potassium, le carbonate de potassium, le sulfate de potassium, le lactate de potassium, le glutamate de potassium, le succinate de potassium, le malate de potassium, le sulfate de sodium, le sulfate de magnésium ; les principes actifs pharmaceutiques, en particulier les antibiotiques de la famille des fluoroquinolones, le paracétamol, l'aspirine, les antibiotiques de la famille des β-lactames, l'ambroxol, le propylthiouracile [PROP], la guaïfénésine ; les vitamines, en particulier la vitamine H, les vitamines de la série B telles que les vitamines B1, B2, B6, B12, la niacine, l'acide panthoténique) ; le benzoate de dénatonium ; l'octaacétate de sucralose ; les sels de fer ; les sels d'aluminium ; les sels de zinc ; l'urée ; les acides gras insaturés, en particulier les acides gras insaturés en émulsions ; les acides aminés ayant un goût amer, en particulier la leucine, l'isoleucine, la valine, le tryptophane, la proline, l'histidine, la tyrosine, la lysine ou la phénylalanine ; et les peptides ayant un goût amer, en particulier les peptides ayant au niveau du site N- ou C-terminal un acide aminé du groupe consistant en la leucine, l'isoleucine, la valine, le tryptophane, la proline ou la phénylalanine.

7. Préparation selon l'une des revendications 4 à 6, comprenant en outre une ou plusieurs lactones, de préférence le nonénolide et/ou la 4-méthyl-5-hydroxyhexanolactone.

8. Préparation selon l'une des revendications 4 à 7, la préparation étant une préparation pharmaceutique orale, une préparation servant à la nutrition, une préparation servant au plaisir ou une préparation servant à l'hygiène buccale.

9. Préparation selon l'une des revendications 4 à 8, comprenant en outre au moins une substance ayant un goût désagréable, la quantité de la substance ayant un goût désagréable suffisant pour être perçue comme correspondant à un goût désagréable dans une préparation comparative qui ne contient ni un dérivé de la 4-hydroxychalcone de formule (I), ni un sel ou un mélange d'un tel dérivé de la 4-hydroxychalcone, mais qui pour le reste a une composition identique, et la quantité du dérivé de la 4-hydroxychalcone de formule (I), du sel ou du mélange d'un tel dérivé de la 4-hydroxychalcone dans la préparation, suffisant pour modifier, masquer ou atténuer d'un point de vue sensoriel, par comparaison avec la préparation comparative, l'impression de goût désagréable de la substance ayant un goût désagréable.

10. Préparation selon l'une des revendications 4 à 9, **caractérisée en ce que** la préparation contient, par rapport à son poids total, en tout 0,000001 à 95 % en poids d'un ou plusieurs dérivés de la 4-hydroxychalcone et, dans la mesure où ils sont présents, de son ou de ses sels.

11. Préparation selon l'une des revendications 4 à 10, **caractérisée en ce qu'**elle se présente sous forme d'un produit semi-fini, d'une composition d'un agent odorant, aromatisant ou de sapidité, ou sous forme d'un mélange de saveurs.

12. Préparation selon l'une des revendications 4 à 11, comprenant en outre au moins une substance supplémentaire pour masquer ou atténuer l'impression de goût désagréable d'une substance ayant un goût désagréable.

13. Procédé pour masquer ou atténuer l'impression de goût désagréable d'une substance ayant un goût désagréable, comprenant l'étape consistant à mélanger une substance ayant un goût désagréable à
- un dérivé de la 4-hydroxychalcone de formule (I) 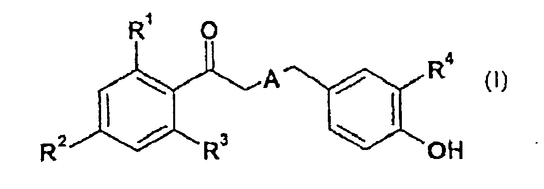 où
A peut être une simple liaison ou une double liaison,
R¹, R² et R³ peuvent représenter chacun indépendamment des autres H, OH ou un groupe alcoxy de préférence en C₁-C₄, à la condition qu'au moins l'un des radicaux R¹ à R³ soit OH,
et R⁴ est H, OH ou un groupe alcoxy de préférence en C₁-C₄,
et/ou
- un sel d'un tel dérivé de la 4-hydroxychalcone de formule (I),
- un mélange comprenant deux dérivés différents, ou plus, de la 4-hydroxychalcone de formule (I), chacun des radicaux R¹, R², R³ et R⁴ ayant les significations données ci-dessus, ou en étant constitué,
- un mélange comprenant des sels de deux dérivés différents, ou plus, de la 4-hydroxychalcone de formule (I), chacun des radicaux R¹, R², R³ et R⁴ ayant les significations ci-dessus, ou en étant constitué,
ou
- un mélange comprenant un dérivé de la 4-hydroxychalcone de formule (I) ou deux dérivés différents, ou plus, de la 4-hydroxychalcone de formule (I), chacun des radicaux R¹, R², R³ et R⁴ ayant les significations données ci-dessus, et un sel d'un dérivé de la 4-hydroxychalcone de formule (I), ou deux sels ou plus de dérivés différents de la 4-hydroxychalcone de formule (I), chacun des radicaux R¹, R², R³ et R⁴ ayant les significations données ci-dessus, ou en étant constitué.

14. Procédé selon la revendication 13, dans lequel
- le dérivé de la 4-hydroxychalcone de formule (I) est choisi dans le groupe consistant en :
la 3-(4-hydroxyphényl)-1-(2-hydroxyphényl)propan-1-one (composé 1)
la 3-(4-hydroxyphényl)-1-(2,4-dihydroxyphényl)propan-1-one (davidigénine, composé 2)
la 3-(4-hydroxyphényl)-1-(2,6-dihydroxyphényl)propan-1-one (composé 3)
la 3-(4-hydroxyphényl)-1-(2,4,6-trihydroxyphényl)propan-1-one (florétine, composé 4)
la 3-(4-hydroxy-3-méthoxyphényl)-1-(2-hydroxyphényl)propan-1-one (composé 5)
la 3-(4-hydroxy-3-méthoxyphényl)-1-(2,4-dihydroxyphényl)propan-1-one (composé 6)
la 3-(4-hydroxy-3-méthoxyphényl)-1-(2,6-dihydroxyphényl)propan-1-one (composé 7)
la 3-(4-hydroxy-3-méthoxyphényl)-1-(2,4,6-trihydroxyphényl)propan-1-one (composé 8)
la 3-(3,4-dihydroxyphényl)-1-(2,4,6-trihydroxyphényl)propan-1-one (3-hydroxyflorétine, composé 9)
la 3-(4-hydroxy-3-méthoxyphényl)-1-(4-hydroxyphényl)propan-1-one (composé 10),
- le sel est un sel d'un tel dérivé de la 4-hydroxychalcone de formule (I),
- le mélange est un mélange comprenant deux dérivés, ou plus, de la 4-hydroxychalcone de formule (I), choisis dans ledit groupe, ou en étant constitué,
- le mélange est un mélange comprenant des sels de deux dérivés, ou plus, de la 4-hydroxychalcone de formule (I), choisis dans ledit groupe, ou en étant constitué,
ou
- le mélange est un mélange comprenant un dérivé de la 4-hydroxychalcone de formule (I), choisi dans ledit groupe, ou deux dérivés, ou plus, de la 4-hydroxychalcone de formule (I), choisis dans ledit groupe, et un sel d'un dérivé de la 4-hydroxychalcone de formule (I) choisi dans ledit groupe, ou deux sels ou plus de dérivés différents de la 4-hydroxychalcone de formule (I), choisis dans ledit groupe, ou en étant constitué.
